# EUROPÄISCHE PATENTSCHRIFT

(11) **EP 0 915 863 B1**
(45) Veröffentlichungstag und Bekanntmachung des Hinweises auf die Patenterteilung: **15.01.2003**
(21) Anmeldenummer: 97930521.6
(22) Anmeldetag: 11.07.1997
(51) Int. Cl.: C07D 307/30, A01N 43/08

(54) **DIHYDROFURAN-CARBOXAMIDE**
DIHYDROFURAN CARBOXAMIDES
CARBOXAMIDES DE DIHYDROFURANE

(30) Priorität: 24.07.1996 DE 19629825
(43) Veröffentlichungstag der Anmeldung: 19.05.1999
(73) Patentinhaber: Bayer CropScience AG, 40789 Monheim (DE)
(72) Erfinder: ELBE, Hans-Ludwig, D-42329 Wuppertal (DE); KUNISCH, Franz, D-51519 Odenthal (DE); BIELEFELDT, Dietmar, D-40883 Ratingen (DE); TIEMANN, Ralf, D-51375 Leverkusen (DE); STENZEL, Klaus, D-40595 Düsseldorf (DE); DUTZMANN, Stefan, D-40764 Langenfeld (DE); KUGLER, Martin, D-42799 Leichlingen (DE); SCHRAGE, Heinrich, D-47829 Krefeld (DE)
(86) Internationale Anmeldenummer: EP9703693
(87) Internationale Veröffentlichungsnummer: WO98003495

(56) Entgegenhaltungen:
- DE-A- 1 914 954
- PATENT ABSTRACTS OF JAPAN vol. 014, no. 417 (C-0756), 10.September 1990 & JP 02 157266 A (MITSUBISHI KASEI CORP), 18.Juni 1990,

## Beschreibung

Die vorliegende Erfindung betrifft neue Dihydrofuran-carboxamide, ein Verfahren zu deren Herstellung und deren Verwendung als Mikrobizide.

Es ist bereits bekannt, daß bestimmte Dihydrofurancarboxanilide fungizide Eigenschaften besitzen (vgl. DE-A 1 914 954 und J. Pesticide Sci. 18 (1993), 245-251). So lassen sich z.B. 2-Methyl-4,5-dihydrofuran-3-carboxanilid und 2-Methyl-4,5-dihydrofuran-3-N-(1,1,3-trimethyl-indan-4-yl)-carboxamid zur Bekämpfung von Pilzen verwenden. Die Wirksamkeit dieser Stoffe ist gut, läßt aber bei niedrigen Aufwandmengen in manchen Fällen zu wünschen übrig.

Es wurden nun neue Dihydrofuran-carboxamide der Formel 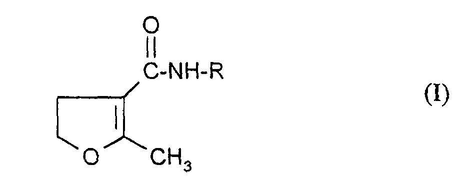 in welcher
- R: für Gruppierungen der Formeln 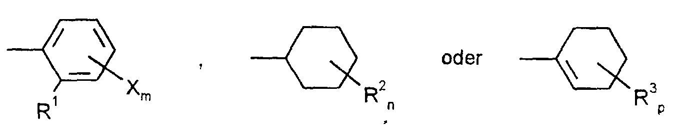 steht, worin
R¹ für gegebenenfalls substituiertes Cycloalkyl, gegebenenfalls substituiertes Bicycloalkyl, gegebenenfalls substituiertes Cycloalkenyl, gegebenenfalls substituiertes Cycloalkylalkyl, gegebenenfalls substituiertes Aryl, gegebenenfalls substituiertes Aroxy, gegebenenfalls substituiertes Aralkyl oder für Alkyl steht,
X für Alkyl mit 1 bis 6 Kohlenstoffatomen, Halogen, Cycloalkyl mit 3 bis 8 Kohlenstoffatomen, Alkoxy mit 1 bis 6 Kohlenstoffatomen, Halogenalkyl mit 1 bis 4 Kohlenstoffatomen und 1 bis 5 Halogenatomen oder Halogenalkoxy mit 1 bis 4 Kohlenstoffatomen und 1 bis 5 Halogenatomen steht,
m für ganze Zahlen von 0 bis 3 steht,
R² für Alkyl, Cycloalkyl, gegebenenfalls substituiertes Aryl oder gegebenenfalls substituiertes Aralkyl steht,
n für ganze Zahlen von 0 bis 3 steht,
R³ für Alkyl, Cycloalkyl, gegebenenfalls substituiertes Aryl oder gegebenenfalls substituiertes Aralkyl steht und
p für ganze Zahlen von 0 bis 3 steht,
gefunden.

Weiterhin wurde gefunden, daß man Dihydrofuran-carboxamide der Formel (I) erhält, wenn man 2-Methyl-4,5-dihydrofuran-3-carbonsäure-halogenide der Formel 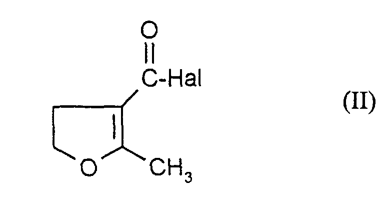 in welcher
- Hal: für Chlor oder Brom steht,
mit Aminen der Formel

H₂N-R (III)

in welcher
- R: die oben angegebenen Bedeutungen hat,
gegebenenfalls in Gegenwart eines Säurebindemittels und gegebenenfalls in Gegenwart eines Verdünnungsmittels umsetzt.

Schließlich wurde gefunden, daß die neuen Dihydrofuran-carboxamide der Formel (I) sehr gute mikrobizide Eigenschaften besitzen und zur Bekämpfung unerwünschter Mikroorganismen sowohl im Pflanzenschutz als auch im Materialschutz eingesetzt werden können.

Überraschenderweise zeigen die erfindungsgemäßen Dihydrofuran-carboxamide eine wesentlich bessere fungizide Wirksamkeit als 2-Methyl-4,5-dihydrofuran-3-carboxanilid und 2-Methyl-4,5-dihydrofuran-3-N-(1,1,3-trimethylindan-4-yl)-carboxamid, welches konstitutionell ähnliche, vorbekannte Wirkstoffe gleicher Wirkungsrichtung sind.

Die erfindungsgemäßen Dihydrofuran-carboxamide sind durch die Formel (I) allgemein definiert.
- R: steht vorzugsweise für die Gruppierungen der Formeln 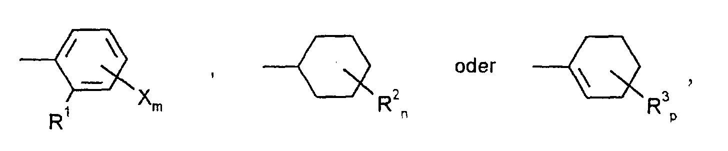 worin
R¹ vorzugsweise für gegebenenfalls einfach bis dreifach, gleichartig oder verschieden durch Alkyl mit 1 bis 4 Kohlenstoffatomen substituiertes Cycloalkyl mit 3 bis 8 Kohlenstoffatomen, für gegebenenfalls einfach bis dreifach, gleichartig oder verschieden durch Alkyl mit 1 bis 4 Kohlenstoffatomen substituiertes Bicycloalkyl mit 7 bis 12 Kohlenstoffatomen, für gegebenenfalls einfach bis dreifach, gleichartig oder verschieden durch Alkyl mit 1 bis 4 Kohlenstoffatomen substituiertes Cycloalkenyl mit 5 bis 8 Kohlenstoffatomen oder für gegebenenfalls einfach bis dreifach, gleichartig oder verschieden durch Alkyl mit 1 bis 4 Kohlenstoffatomen substituiertes Cycloalkylalkyl mit 3 bis 8 Kohlenstoffatomen im Cycloalkylteil und 1 bis 4 Kohlenstoffatomen im Alkylteil steht, oder
für Phenyl steht, das einfach bis dreifach, gleichartig oder verschieden durch Halogen und/oder Alkyl mit 1 bis 4 Kohlenstoffatomen substituiert sein kann, oder
für Phenoxy steht, das einfach bis dreifach, gleichartig oder verschieden durch Halogen und/oder Alkyl mit 1 bis 4 Kohlenstoffatomen substituiert sein kann, oder
für Phenylalkyl mit 1 bis 4 Kohlenstoffatomen im Alkylteil steht, das im Phenylteil einfach bis dreifach, gleichartig oder verschieden durch Halogen und/oder Alkyl mit 1 bis 4 Kohlenstoffatomen substituiert sein kann,
oder für geradkettiges oder verzweigtes Alkyl mit 1 bis 12 Kohlenstoffatomen steht,
X vorzugsweise für geradkettiges oder verzweigtes Alkyl mit 1 bis 4 Kohlenstoffatomen, Fluor, Chlor, Brom, Cycloalkyl mit 3 bis 8 Kohlenstoffatomen, geradkettiges oder verzweigtes Alkoxy mit 1 bis 4 Kohlenstoffatomen, Halogenalkyl mit 1 oder 2 Kohlenstoffatomen und 1 bis 5 Fluor-, Chlor- und/oder Bromatomen oder für Halogenalkoxy mit 1 oder 2 Kohlenstoffatomen und 1 bis 5 Fluor-, Chlor- und/oder Bromatomen steht,
m vorzugsweise für die Zahlen 0, 1 oder 2 steht,
R² vorzugsweise für geradkettiges oder verzweigtes Alkyl mit 1 bis 12 Kohlenstoffatomen, Cycloalkyl mit 3 bis 8 Kohlenstoffatomen, gegebenenfalls einfach bis dreifach, gleichartig oder verschieden durch Halogen und/oder Alkyl mit 1 bis 4 Kohlenstoffatomen substituiertes Phenyl oder für gegebenenfalls einfach bis dreifach, gleichartig oder verschieden durch Halogen und/oder Alkyl mit 1 bis 4 Kohlenstoffatomen substituiertes Phenylalkyl mit 1 bis 4 Kohlenstoffatomen im Alkylteil steht,
n vorzugsweise für die Zahlen 0, 1, 2 oder 3 steht,
R³ vorzugsweise für geradkettiges oder verzweigtes Alkyl mit 1 bis 12 Kohlenstoffatomen, Cycloalkyl mit 3 bis 8 Kohlenstoffatomen, gegebenenfalls einfach bis dreifach, gleichartig oder verschieden durch Halogen und/oder Alkyl mit 1 bis 4 Kohlenstoffatomen substituiertes Phenyl oder für gegebenenfalls einfach bis dreifach, gleichartig oder verschieden durch Halogen und/oder Alkyl mit 1 bis 4 Kohlenstoffatomen substituiertes Phenylalkyl mit 1 bis 4 Kohlenstoffatomen im Alkylteil steht und
p vorzugsweise für die Zahlen 0, 1, 2 oder 3 steht.

Besonders bevorzugt sind Dihydrofuran-carboxamide der Formel (I), in denen
- R: für eine Gruppierung der Formel 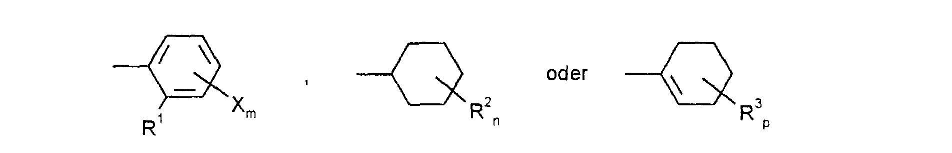 steht, worin
R¹ für gegebenenfalls einfach bis dreifach, gleichartig oder verschieden durch Methyl, Ethyl, n-Propyl, Isopropyl und/oder tert.-Butyl substituiertes Cycloalkyl mit 3 bis 8 Kohlenstoffatomen steht, oder für gegebenenfalls einfach bis dreifach, gleichartig oder verschieden durch Methyl, Ethyl, n-Propyl, Isopropyl und/oder tert.-Butyl substituiertes Bicycloalkyl mit 7 bis 12 Kohlenstoffatomen steht, oder
für gegebenenfalls einfach bis dreifach, gleichartig oder verschieden durch Methyl, Ethyl, n-Propyl, Isopropyl und/oder tert.-Butyl substituiertes Cycloalkenyl mit 5 bis 8 Kohlenstoffatomen steht, oder für gegebenenfalls einfach bis dreifach, gleichartig oder verschieden durch Methyl, Ethyl, n-Propyl, Isopropyl und/oder tert.-Butyl substituiertes Cycloalkylalkyl mit 3 bis 8 Kohlenstoffatomen im Cycloalkylteil und 1 oder 2 Kohlenstoffatomen im Alkylteil steht, oder
für Phenyl steht, das einfach bis dreifach, gleichartig oder verschieden durch Fluor, Chlor, Brom, Methyl, Ethyl, n-Propyl, Isopropyl und/oder tert.-Butyl substituiert sein kann, oder
für Phenoxy steht, das einfach bis dreifach, gleichartig oder verschieden durch Fluor, Chlor, Brom, Methyl, Ethyl, n-Propyl, Isopropyl und/oder tert.-Butyl substituiert sein kann, oder
für Phenylalkyl mit 1 oder 2 Kohlenstoffatomen im Alkylteil steht, das im Phenylteil einfach bis dreifach, gleichartig oder verschieden durch Fluor, Chlor, Brom, Methyl, Ethyl, n-Propyl, Isopropyl und/oder tert.-Butyl substituiert sein kann, oder
für geradkettiges oder verzweigtes Alkyl mit 1 bis 12 Kohlenstoffatomen steht,
X für Methyl, Ethyl, n-Propyl, Isopropyl, tert.-Butyl, Fluor, Chlor, Brom, Cyclopropyl, Cyclopentyl, Cyclohexyl, Cycloheptyl, Cyclooctyl, Methoxy, Ethoxy, Trichlormethyl, Trifluormethyl, Difluormethyl, Difluorchlormethyl, Trifluormethoxy oder Difluormethoxy steht,
m für die Zahlen 0, 1 oder 2 steht, wobei X für gleiche oder verschiedene Reste steht, wenn m für 2 steht,
R² für Methyl, Ethyl, n-Propyl, Isopropyl, n-Butyl, sek.-Butyl, iso-Butyl, tert.-Butyl, 2-Ethyl-butyl, Octyl, Decyl, Dodecyl, Cyclopropyl, Cyclopentyl, Cyclohexyl, Cycloheptyl, Cyclooctyl, gegebenenfalls einfach oder zweifach durch Fluor, Chlor, Brom, Methyl, Ethyl, n-Propyl, Isopropyl und/oder tert.-Butyl substituiertes Phenyl oder für Phenylalkyl mit 1 oder 2 Kohlenstoffatomen im Alkylteil steht, wobei der Phenylteil einfach oder zweifach durch Fluor, Chlor, Brom, Methyl, Ethyl, n-Propyl, Isopropyl und/oder tert.-Butyl substituiert sein kann,
n für die Zahlen 0, 1, 2 oder 3 steht, wobei R² für gleiche oder verschiedene Reste steht, wenn n für 2 oder 3 steht,
R³ für Methyl, Ethyl, n-Propyl, Isopropyl, n-Butyl, sek.-Butyl, iso-Butyl, tert.-Butyl, 2-Ethyl-butyl, Octyl, Decyl, Dodecyl, Cyclopropyl, Cyclopentyl, Cyclohexyl, Cycloheptyl, Cyclooctyl, gegebenenfalls einfach oder zweifach durch Fluor, Chlor, Brom, Methyl, Ethyl, n-Propyl, Isopropyl und/oder tert.-Butyl substituiertes Phenyl oder für Phenylalkyl mit 1 oder 2 Kohlenstoffatomen im Alkylteil steht, wobei der Phenylteil einfach oder zweifach durch Fluor, Chlor, Brom, Methyl, Ethyl, n-Propyl, Isopropyl und/oder tert.-Butyl substituiert sein kann und
p für die Zahlen 0, 1, 2 oder 3 steht, wobei R³ für gleiche oder verschiedene Reste steht, wenn p für 2 oder 3 steht.

Verwendet man 2-Methyl-4,5-dihydrofuran-3-carbonsäurechlorid und 4-Fluor-2-cyclooctyl-anilin als Ausgangsstoffe, so kann der Verlauf des erfindungsgemäßen Verfahrens durch das folgende Formelschema veranschaulicht werden.

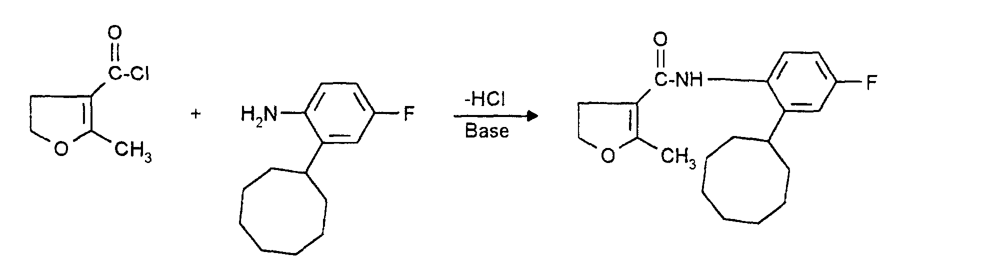

Die bei der Durchführung des erfindungsgemäßen Verfahrens als Ausgangsstoffe benötigten 2-Methyl-4,5-dihydrofuran-3-carbonsäure-halogenide sind durch die Formel (II) allgemein definiert. Hal steht auch vorzugsweise für Chlor oder Brom.

Die 2-Methyl-4,5-dihydrofuran-3-carbonsäure-halogenide sind bekannt oder lassen sich nach bekannten Methoden herstellen (vgl. DE-A 1 914 954 und Chem. Ber. 104 (1971), 734-738).

Die bei der Durchführung des erfindungsgemäßen Verfahrens als Reaktionskomponenten benötigten Amine sind durch die Formel (III) allgemein definiert. In dieser Formel hat R vorzugsweise diejenigen Bedeutungen, die bereits im Zusammenhang mit der Beschreibung der erfindungsgemäßen Stoffe der Formel (I) für diesen Rest als bevorzugt genannt wurden.

Die Amine der Formel (III) sind bekannt oder lassen sich nach bekannten Verfahren herstellen (vgl. US-A 5 223 526, EP-A 0 545 099, EP-A 0 589 301 und DE-A 44 45 545).

Als Säurebindemittel kommen bei der Durchführung des erfindungsgemäßen Verfahrens alle für derartige Reaktionen üblichen anorganischen und organischen Basen in Betracht. Vorzugsweise verwendbar sind Erdalkali- oder Alkalimetallhydroxide, wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU). Es ist jedoch auch möglich, ohne zusätzliches Säurebindemittel zu arbeiten, oder die Aminkomponente in einem Überschuß einzusetzen, so daß sie gleichzeitig als Säurebindemittel fungiert.

Als Verdünnungsmittel kommen bei der Durchführung des erfindungsgemäßen Verfahrens alle üblichen inerten, organischen Solventien in Frage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methylt-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester; Sulfoxide, wie Dimethylsulfoxid oder Sulfone, wie Sulfolan.

Die Reaktionstemperaturen können bei der Durchführung des erfindungsgemäßen Verfahrens in einem größeren Bereich variiert werden. Im allgemeinen arbeitet man bei Temperaturen zwischen 0°C und 100°C, vorzugsweise zwischen 10°C und 80°C.

Bei der Durchführung des erfindungsgemäßen Verfahrens arbeitet man im allgemeinen unter Atmosphärendruck. Es ist aber auch möglich, unter erhöhtem oder vermindertem Druck zu arbeiten.

Bei der Durchführung des erfindungsgemäßen Verfahrens setzt man auf 1 Mol an 2-Methyl-4,5-dihydrofuran-3-carbonsäure-halogenid der Formel (II) im allgemeinen 1 Mol oder auch einen Überschuß an Amin der Formel (III) sowie 1 bis 3 Mol an Säurebindemittel ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, daß man das Reaktionsgemisch mit Wasser versetzt, die organische Phase abtrennt und nach dem Trocknen unter vermindertem Druck einengt. Der verbleibende Rückstand kann gegebenenfalls nach üblichen Methoden, wie Chromatographie oder Umkristallisation, von eventuell noch vorhandenen Verunreinigungen befreit werden.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst- und Gemüseanbau, wie beispielsweise gegen Venturia-, Podosphaera-, Phytophtora- und Plasmopara-Arten, einsetzen. Mit gutem Erfolg werden auch Reiskrankheiten, wie beispielsweise Pyricularia-Arten, bekämpft.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat(Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilate, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofosmethyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
2',6'-Dibrom-2-methyl-4'-trifluormethoxy-4'-trifluor-methyl-1,3-thiazol-5-carboxanilid,
2,6-Dichlor-N-(4-trifluormethylbenzyl)-benzamid,
2-Aminobutan,
2-Phenylphenol(OPP),
8-Hydroxychinolinsulfat,
cis-1-(4-Chlorphenyl)-2-(1H-1,2,4-triazol-1-yl)-cycloheptanol,
(5RS,6RS)-6-Hydroxy-2,2,7,7-tetramethyl-5-(1H-1,2,4-triazol-1-yl)-3-octanon,
α-(2,4-Dichlorphenyl)-β-methoxy-α-methyl-1H-1,2,4-triazol-1-ethanol,
α-(1,1-Dimethylethyl)-β-(2-phenoxyethyl)-1H-1,2,4-triazol-1-ethanol,
1-[1-[2-[(2,4-Dichlorphenyl)-methoxy]-phenyl]-ethenyl]-1H-imidazol,
bis-(1-Methylethyl)-3-methyl-4-[(3-methylbenzoyl)-oxy]-2,5-thiophendicarboxylat,
2,6-Dichlor-N-[[4-(trifluormethyl)-phenyl]-methyl]-benzamid,
(E)-α-(Methoxyimino)-N-methyl-2-phenoxy-phenylacetamid,
9H-Xanthen-9-carbonsäure-2-[(phenylamino)-carbonyl]-hydrazid,
O-Methyl-S-phenyl-phenylpropylphosphoramidothioat,
N-(5-Chlor-2-methylphenyl)-2-methoxy-N-(2-oxo-3-oxazolidinyl)-acetamid,
1-(2,4-Dichlorphenyl)-2-(1H-1,2,4-triazol-1-yl)ethanon-O-(phenylmethyl)-oxim,
N-(2,6-Dimethylphenyl)-2-methoxy-N-(tetrahydro-2-oxo-3-thienyl)-acetamid,
cis-4-[3-[4-(1,1-Dimethylpropyl)-phenyl-2-methylpropyl]-2,6-dimethyl-morpholinhydrochlorid,
1-(3,5-Dichlorphenyl)-3-(2-propenyl)-2,5-pyrrolidindion,
1-Methyl-5-nonyl-2-(phenylmethyl)-3-pyrrolidinol,
1-[[2-(4-Chlorphenyl)-3-phenyloxiranyl]-methyl]-1H-1,2,4-triazol,
Methantetrathi ol-Natriumsalz,
2-(2,3,3-Triiod-2-propenyl)-2H-tetrazol,
N-[3-Chlor-4,5-bis(2-propinyloxy)-phenyl]-N'-methoxy-methanimidamid,
α-(5-Methyl-1,3-dioxan-5-yl)-β-[[4-(trifluormethyl)-phenyl]-methylen]-1H-1,2,4-triazol-1-ethanol,
1-(2-Methyl-1-naphthalenyl)-1H-pyrrol-2,5-dion,
N-(2,6-Dimethylphenyl)-2-methoxy-N-(tetrahydro-2-oxo-3-furanyl)-acetamid,
3,4-Dichlor-1-[4-(difluormethoxy)-phenyl]-1H-pyrrol-2,5-dion,
N-[2,2,2-Trichlor-1-[(chloracetyl)-amino]-ethyl]-benzamid,
N-Formyl-N-hydroxy-DL-alanin-Natriumsalz,
N-(4-Cyclohexylphenyl)-1,4,5,6-tetrahydro-2-pyrimidinamin,
4-Methyl-tetrazolo[1,5-a]quinazolin-5(4H)-on,
2-Chlor-N-(2,6-dimethylphenyl)-N-(isothiocyanatomethyl)-acetamid,
Ethyl-[(4-chlorphenyl)-azo]-cyanoacetat,
N-(4-Hexylphenyl)-1,4,5,6-tetrahydro-2-pyrimidinamin,
N-(2-Chlor-4-nitrophenyl)-4-methyl-3-nitro-benzolsulfonamid,
Methyl-N-(chloracetyl)-N-(2,6-dimethylphenyl)-DL-alaninat,
3-[2-(4-Chlorphenyl)-5-ethoxy-3-isoxazolidinyl]-pyridin,
2-[(1-Methylethyl)sulfonyl]-5-(tri chlormethyl)-1,3,4-thiadiazol,
spiro[2H]-1-Benzopyran-2,1'(3'H)-isobenzofuran]-3'-on,
Methyl-N-(2,6-dimethylphenyl)-N-(5-isoxazolylcarbonyl)-DL-alaninat,
Kaliumhydrogencarbonat,
1-[[2-(2,4-Dichlorphenyl)-1,3-dioxolan-2-yl]-methyl]-1H-imidazol,
1-[(Diiodmethyl)-sulfonyl]-4-methyl-benzol,
2-Brom-2-(brommethyl)-pentandinitril,
2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
Methyl-1-(2,3-dihydro-2,2-dimethyl-1H-inden-1-yl)-1H-imidazol-5-carboxylat,
2-Chlor-N-(2,3-dihydro-1,1,3-trimethyl-1H-inden-4-yl)-3-pyridincarboxamid,
O,O-Diethyl-[2-(dipropylamino)-2-oxoethyl]-ethylphosphoramidothioat,
α-(2,4-Dichlorphenyl)-β-fluor-β-propyl-1H-1,2,4-triazol-1-ethanol,
3-(1,1-Dimethylpropyl-1-oxo-1H-inden-2-carbonitril,
2,6-Dichlor-5-(methylthio)-4-pyrimidinyl-thiocyanat,
S-Methyl-1,2,3-benzothiadiazol-7-carbothioat,
N-(6-Methoxy)-3-pyridinyl)-cyclopropancarboxamid,
3,5-Dichlor-N-[cyan-[(1-methyl-2-propynyl)-oxy]-methyl]-benzamid,
4-Chlor-2-cyan-N,N-dimethyl-5-(4-methylphenyl)-1H-imidazol-1-sulfonamid,
8-(1,1-Dimethylethyl)-N-ethyl-N-propyl-1,4-dioxaspiro[4,5]decan-2-methanamin,
2,2-Dichlor-N-[1-(4-chlorphenyl)ethyl]-1-ethyl-3-methyl-cyclopropancarboxamid,
N-(2,3-Dichlor-4-hydroxyphenyl)-1-methyl-cyclohexancarboxamid.

### Bakterizide:

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die zum Schutz technischer Materialien verwendeten Mittel enthalten die Wirkstoffe im allgemeinen in einer Menge von 1 bis 95%, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gewichts-%, vorzugsweise von 0,05 bis 1,0 Gewichts-% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

Die Herstellung und die Verwendung der erfindungsgemäßen Stoffe gehen aus den folgenden Beispielen hervor.

### Herstellungsbeispiele

### Beispiel 1

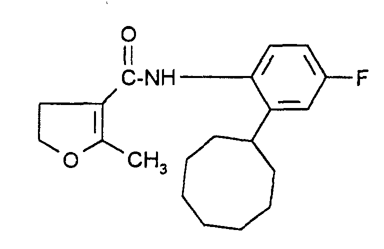

Eine Lösung von 1,5 g (0,01 Mol) 2-Methyl-4,5-dihydrofuran-3-carbonsäurechlorid in 10 ml Toluol wird unter Rühren bei Raumtemperatur in ein Gemisch aus 2,2 g (0,01 Mol) 4-Fluor-2-cyclooctylanilin, 1,0 g Triethylamin und 40 ml Toluol eingetropft. Nach beendeter Zugabe wird noch 2 Stunden bei Raumtemperatur nachgerührt. Anschließend wird das Reaktionsgemisch mit Wasser versetzt. Die organische Phase wird abgetrennt, über Natriumsulfat getrocknet und dann unter vermindertem Druck eingeengt. Der verbleibende Rückstand wird mit Hexan verrührt. Das dabei anfallende kristalline Produkt wird abgesaugt und bei 50°C unter vermindertem Druck getrocknet. Man erhält auf diese Weise 2,2 g (66,7 % der Theorie) an 2-Methyl-4,5-dihydrofuran-3-carbonsäure-(4-fluor-2-cyclooctyl)-anilid in Form einer Festsubstanz vom Schmelzpunkt 91°C.

In analoger Weise werden die in der folgenden Tabelle aufgeführten Dihydrofuran-carboxamide der Formel (I) hergestellt.

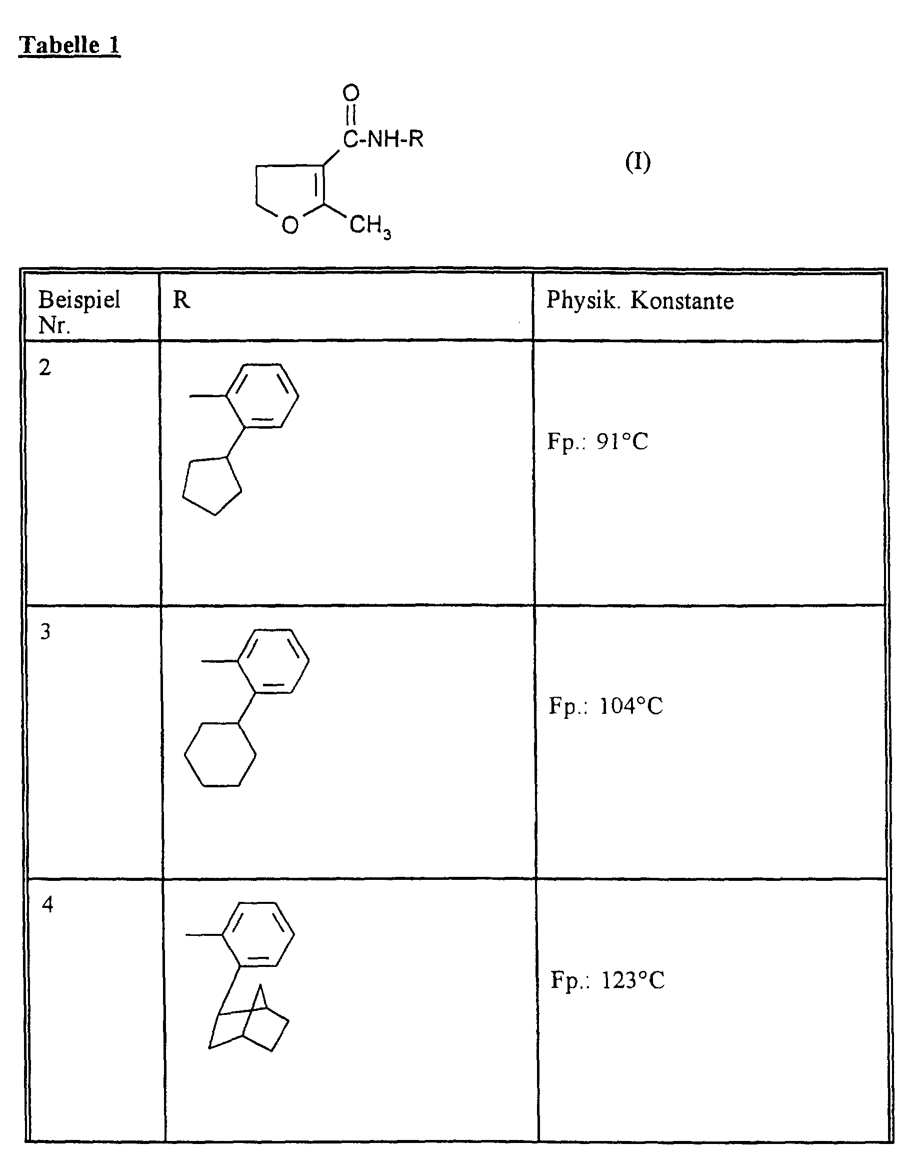

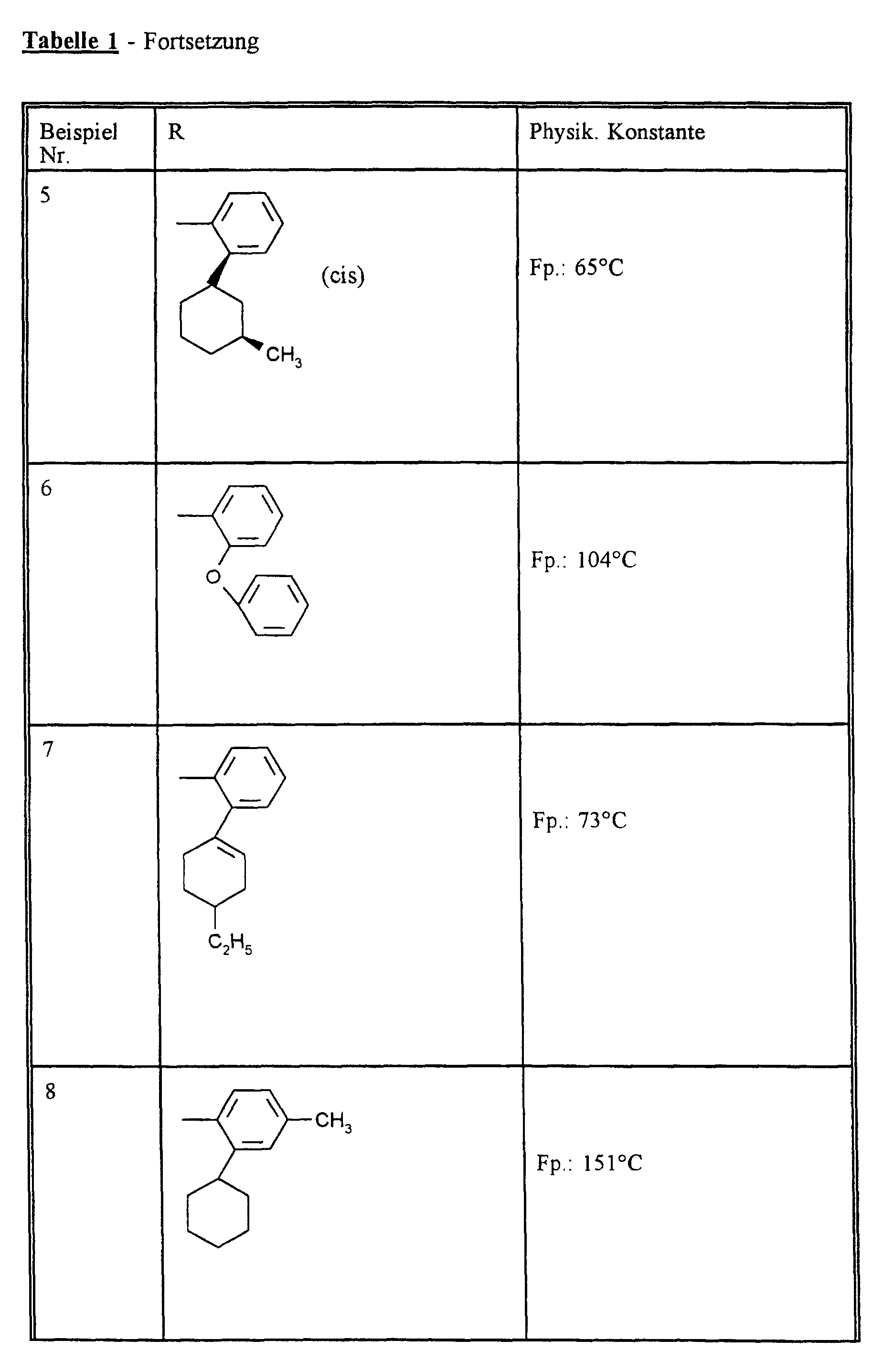

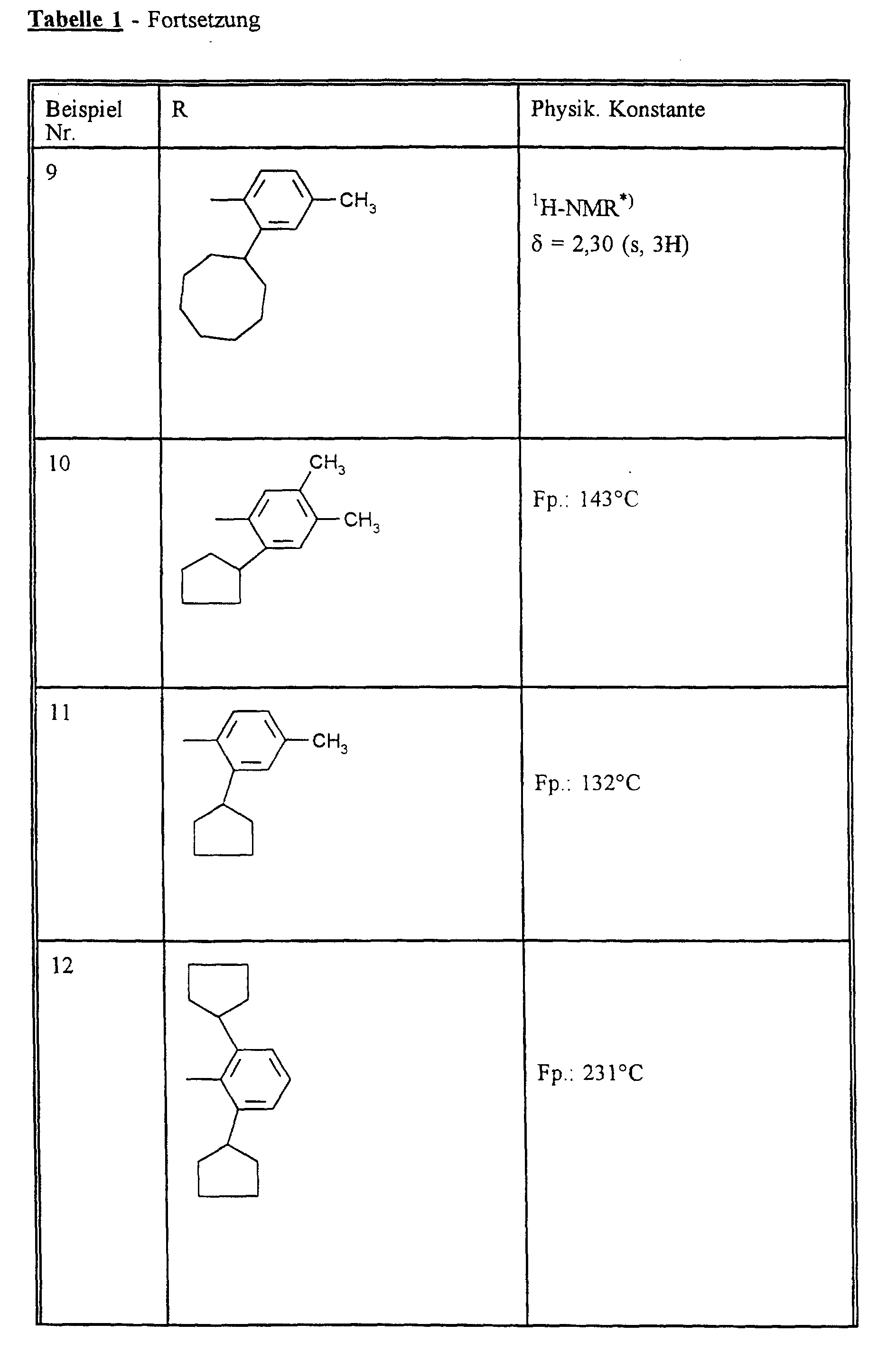

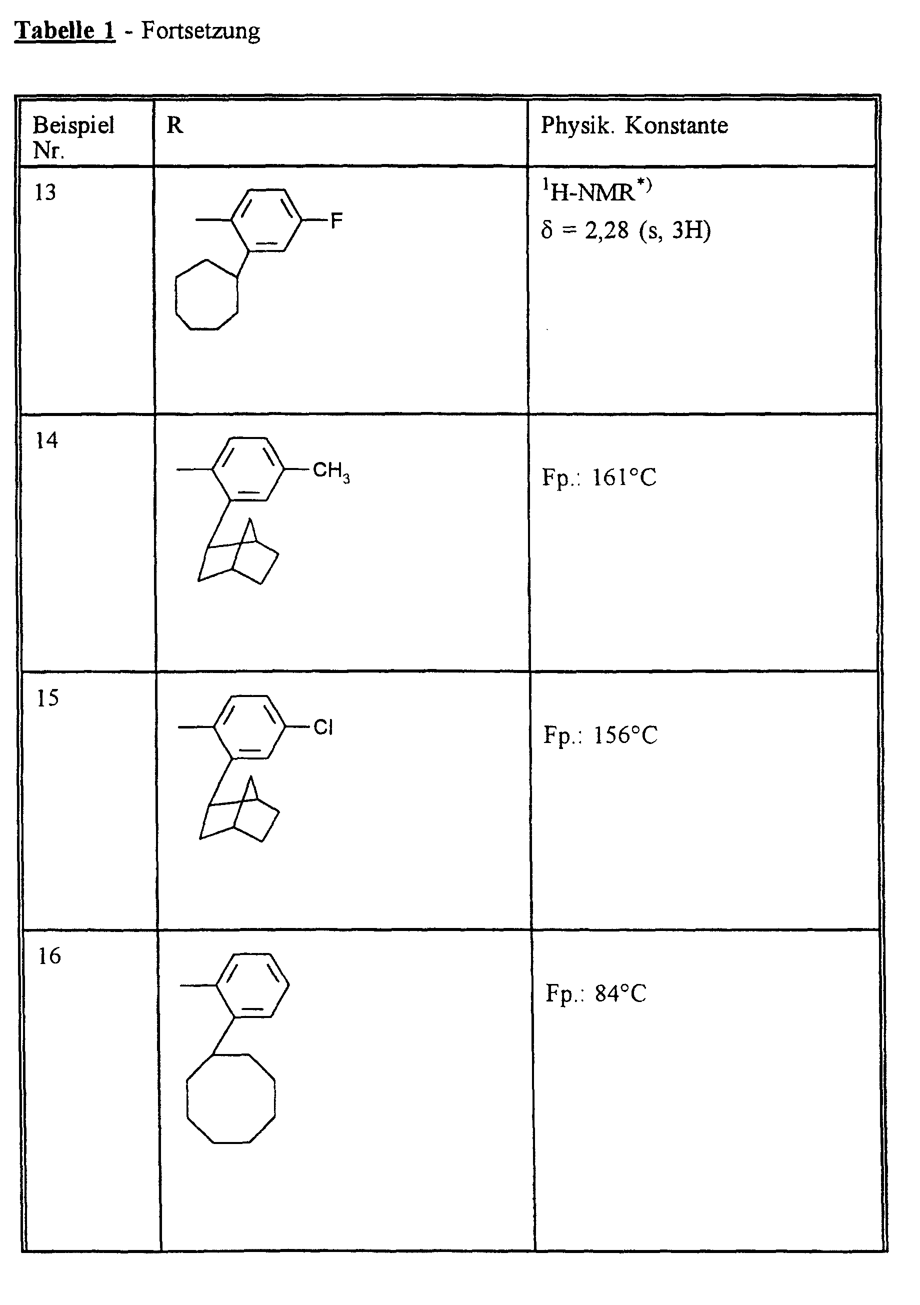

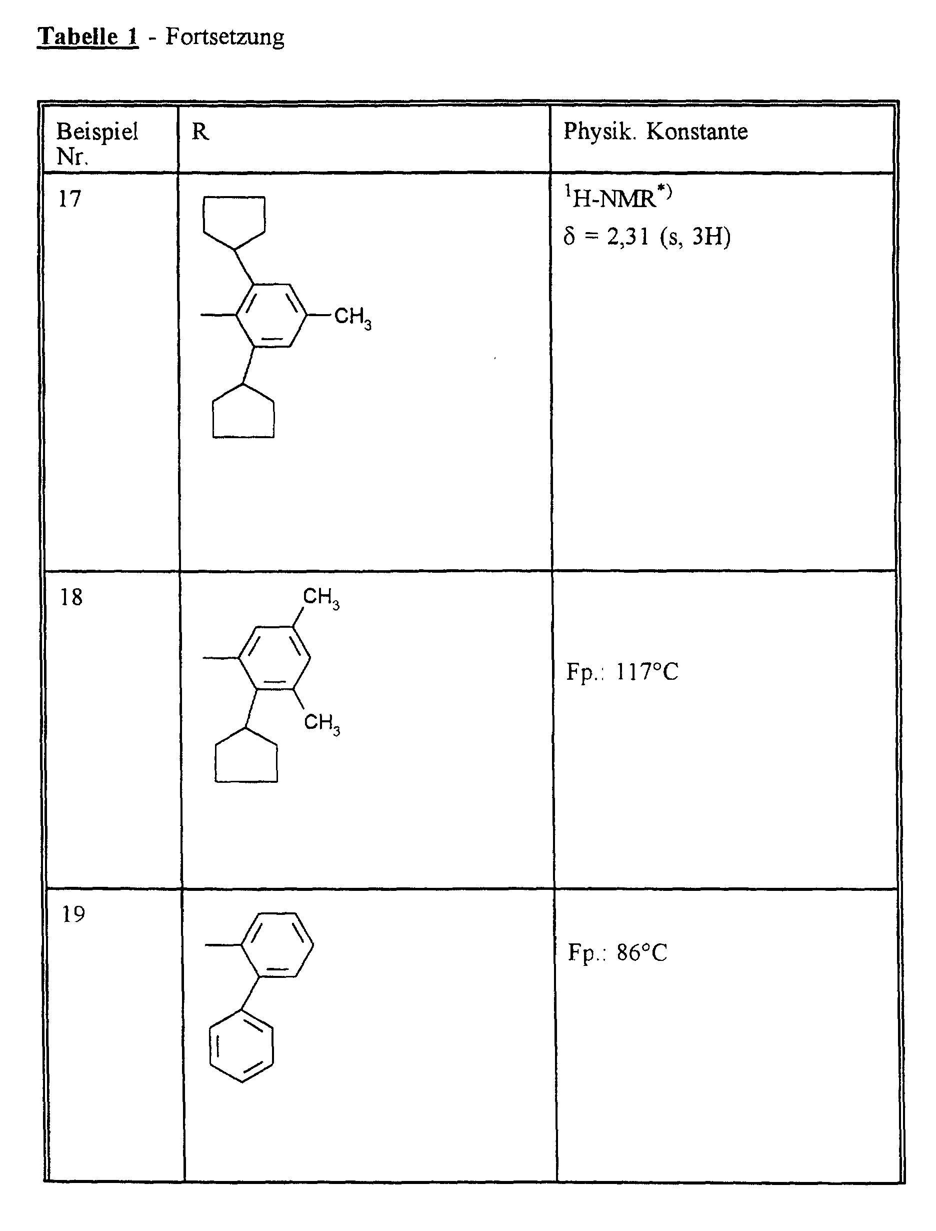

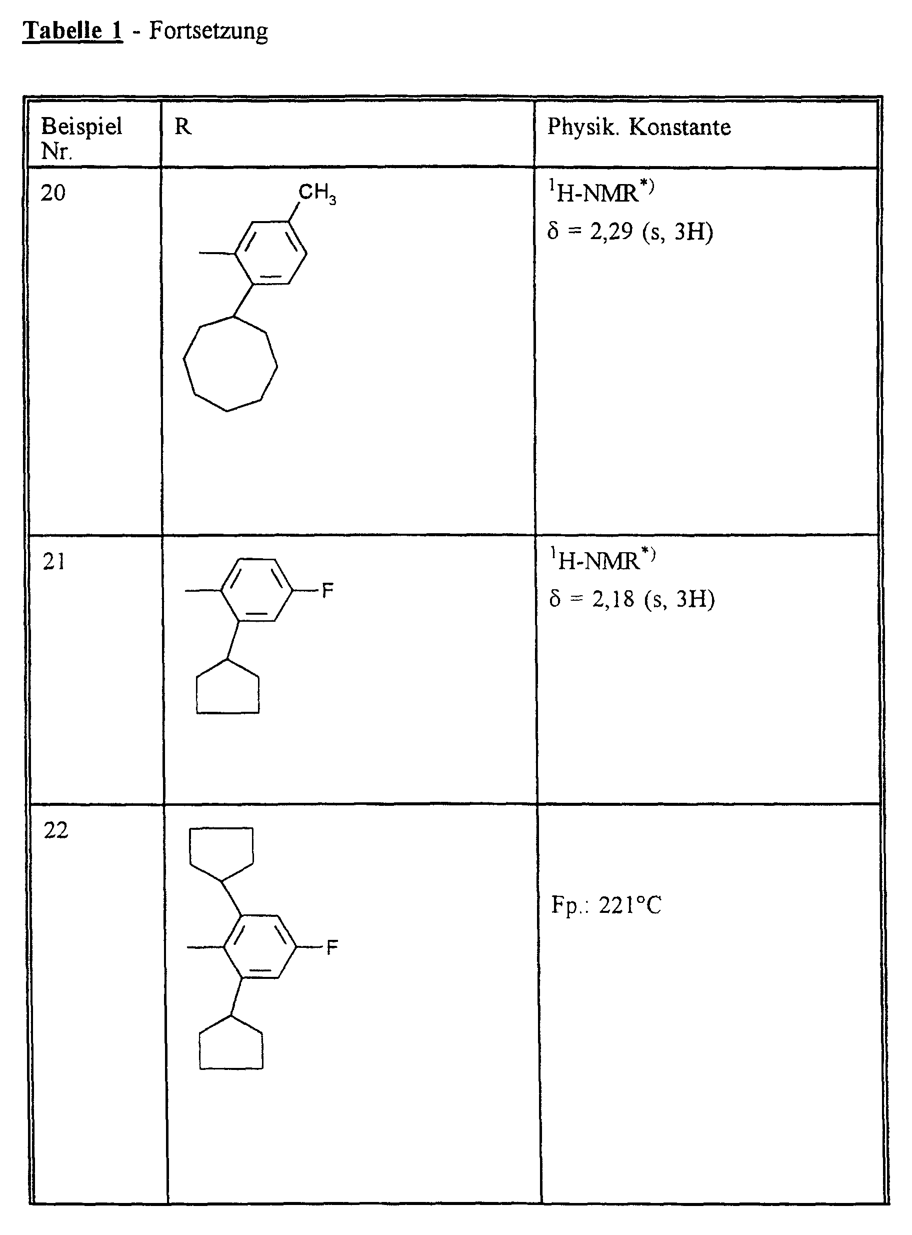

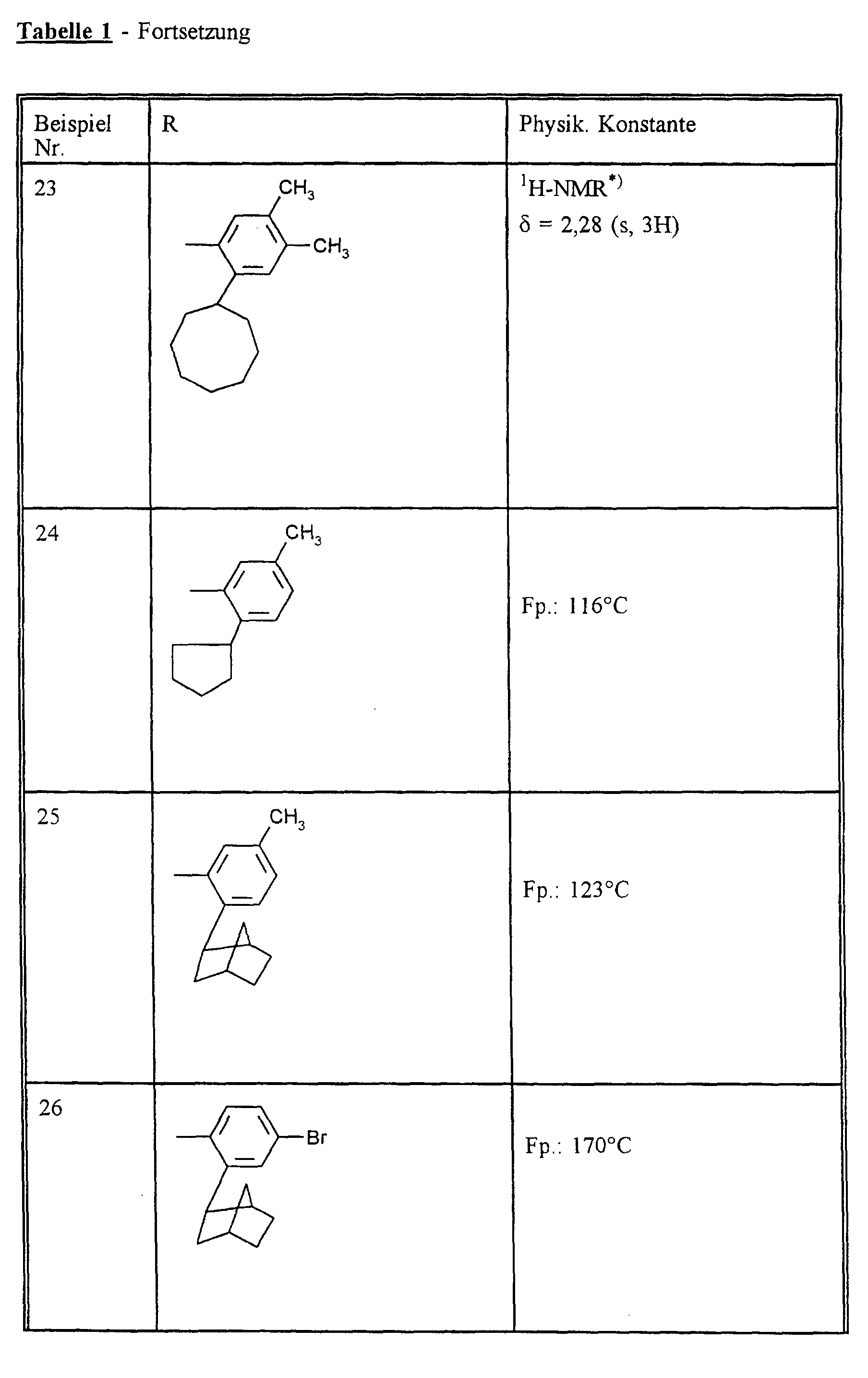

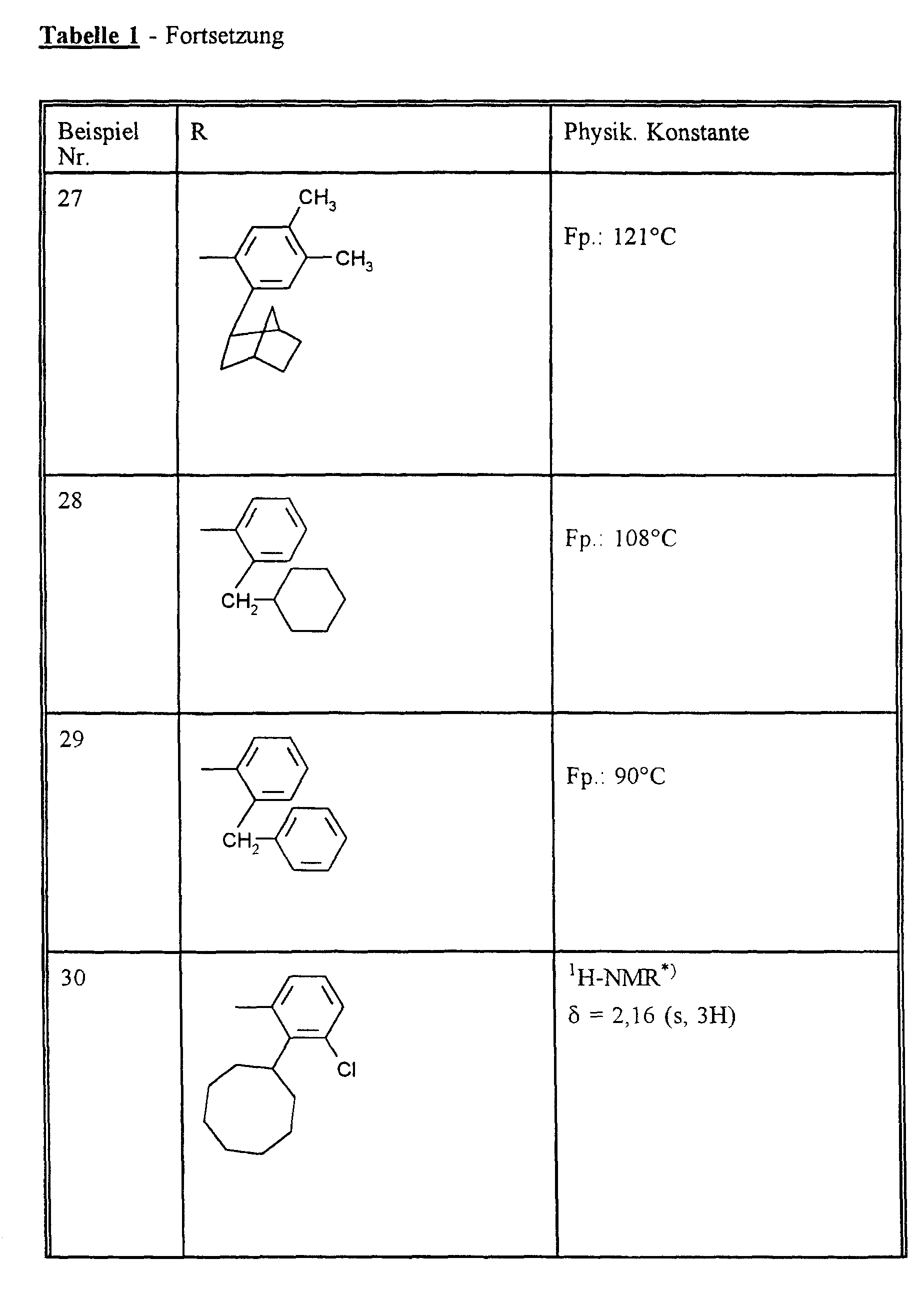

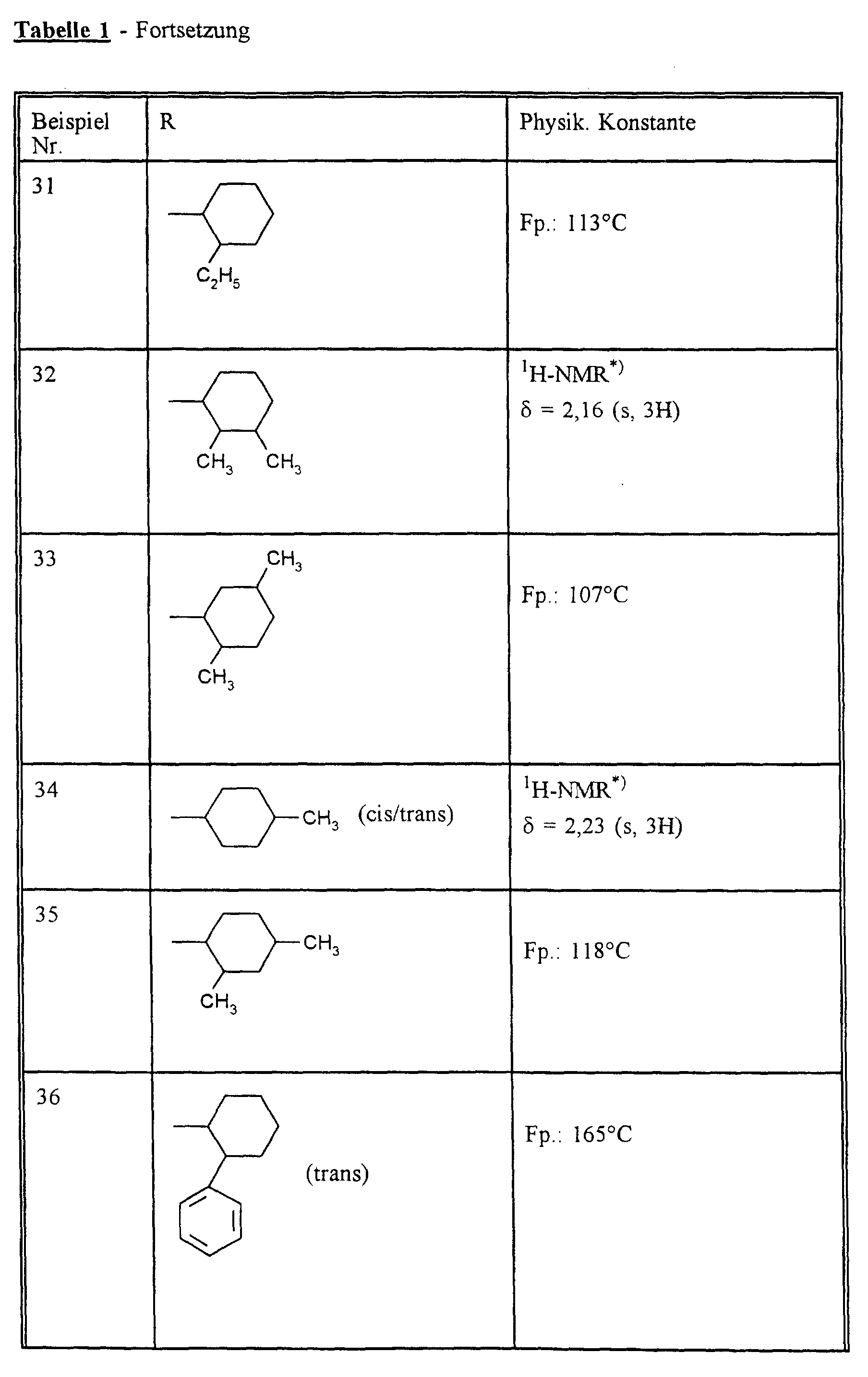

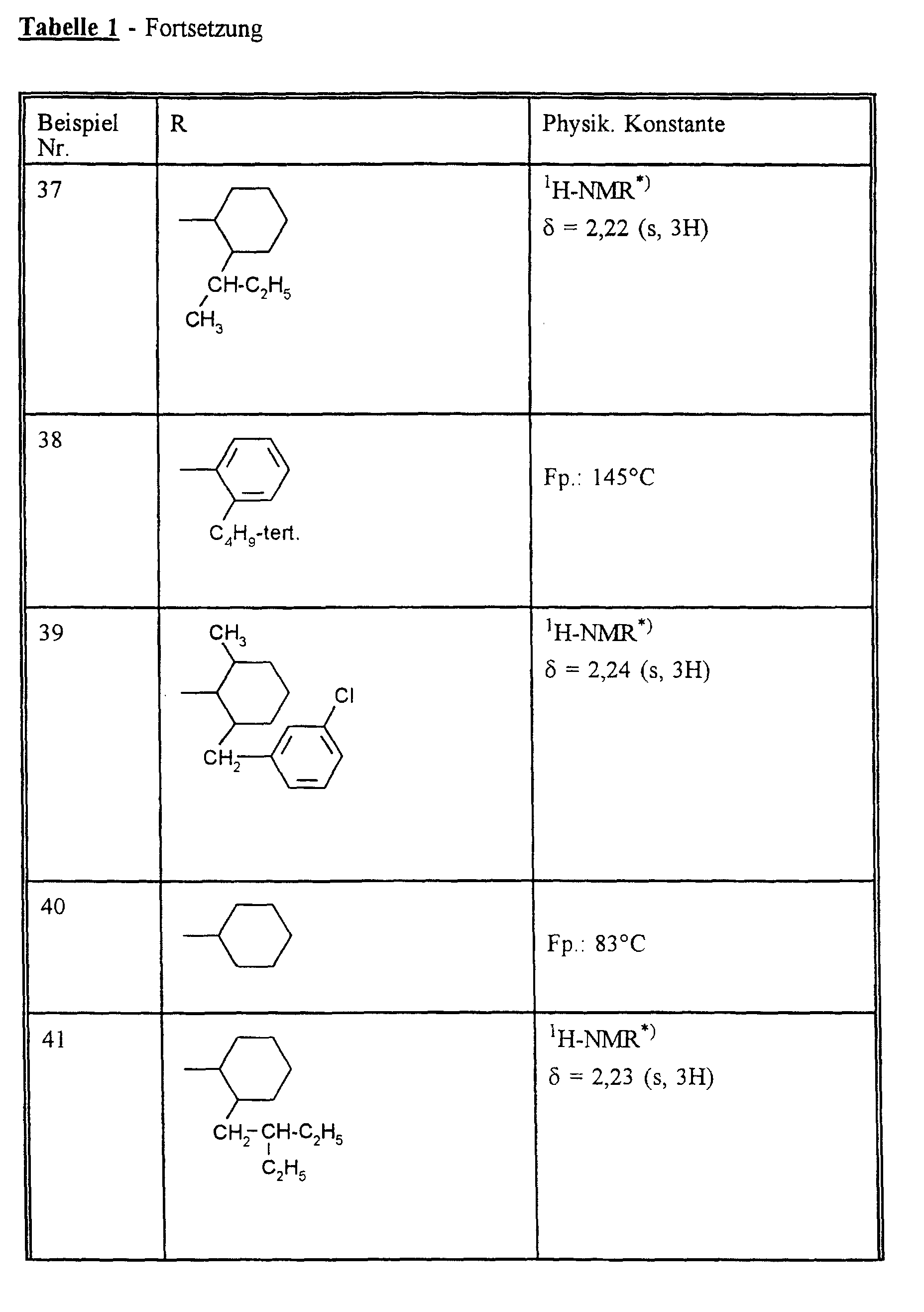

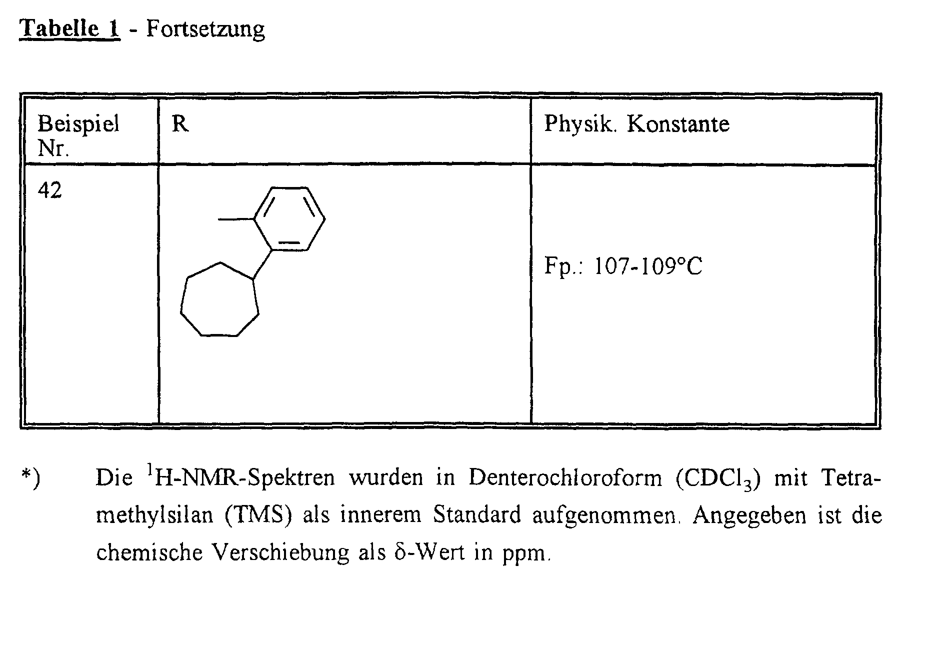

### Verwendungsbeispiele

In den folgenden Beispielen wurden die nachstehend aufgeführten Stoffe als Vergleichssubstanzen eingesetzt:
(A): 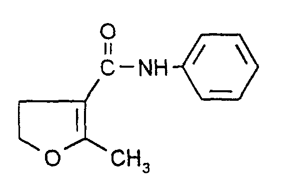 2-Methyl-4,5-dihydrofuran-3-carboxanilid
   (Bekannt aus DE-A 1 914 954)
(B): 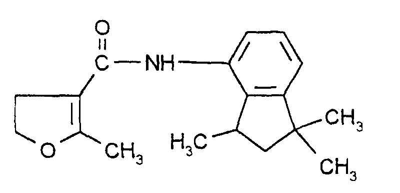 2-Methyl-4,5-dihydrofuran-3-N-(1,1,3-trimethylindan-4-yl)-carboxamid (Bekannt aus J. Pesticide Sci. 18 (1993), 245-251)

### Beispiel A

Botrytis-Test (Bohne) / protektiv

| | |
|---|---|
| Lösungsmittel | 47 Gewichtsteile Aceton |
| Emulgator | 3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden auf jedes Blatt 2 kleine, mit Botrytis cinerea bewachsene Agarstückchen aufgelegt. Die inokulierten Pflanzen werden in einer abgedunkelten, feuchten Kammer bei 20°C aufgestellt.

3 Tage nach der Inokulation wird die Größe der Befallsflecken auf den Blättern ermittelt und in % ausgedrückt. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchergebnisse gehen aus der folgenden Tabelle hervor.

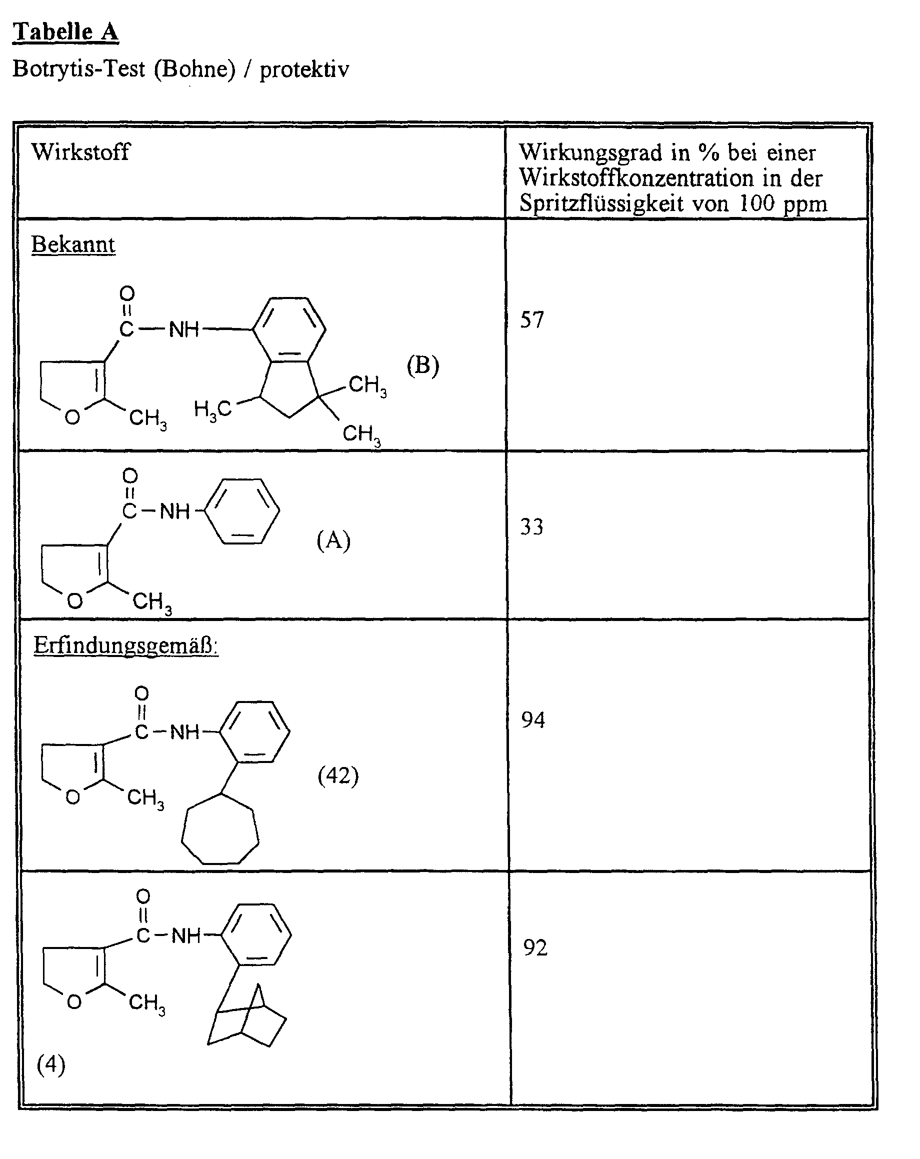

### Beispiel B

Erysiphe-Test (Weizen) / protektiv

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. tritici bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchergebnisse gehen aus der folgenden Tabelle hervor.

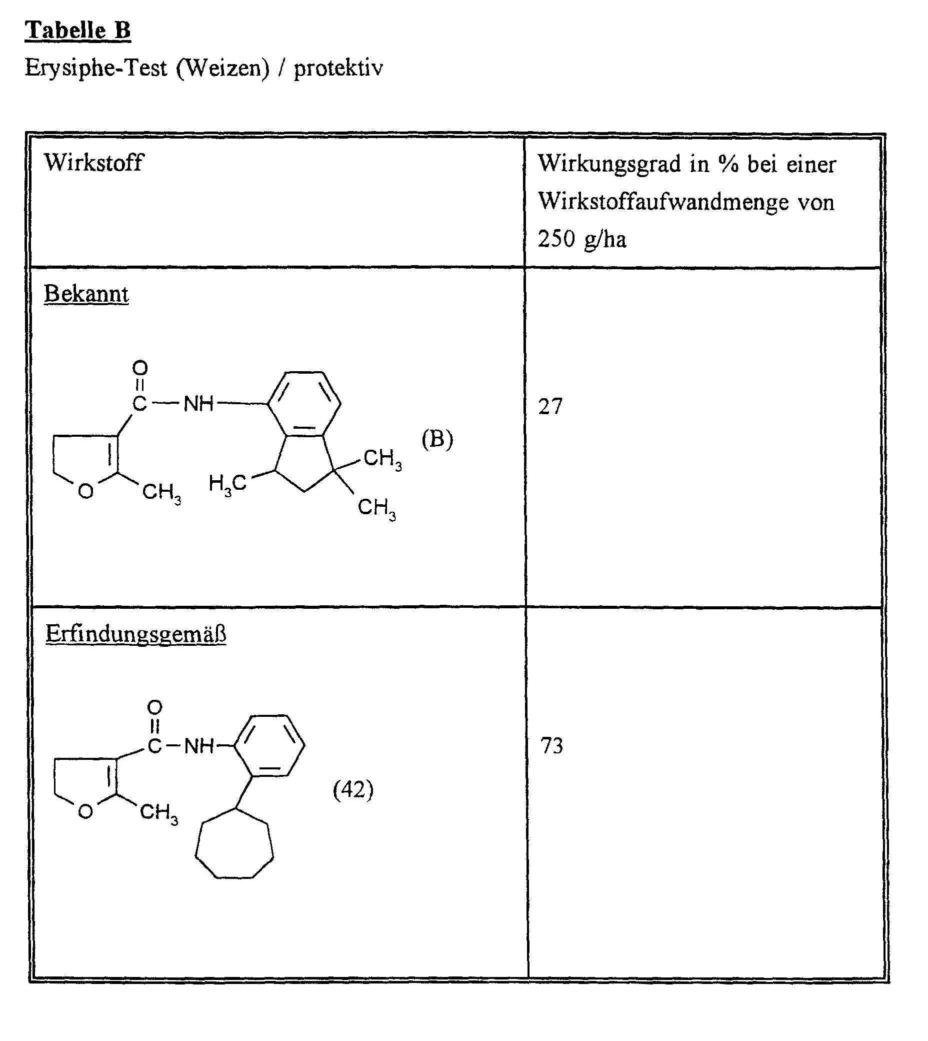

### Beispiel C

Sphaerotheca-Test (Gurke) / protektiv

| | |
|---|---|
| Lösungsmittel | 47 Gewichtsteile Aceton |
| Emulgator | 3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchergebnisse gehen aus der folgenden Tabelle hervor.

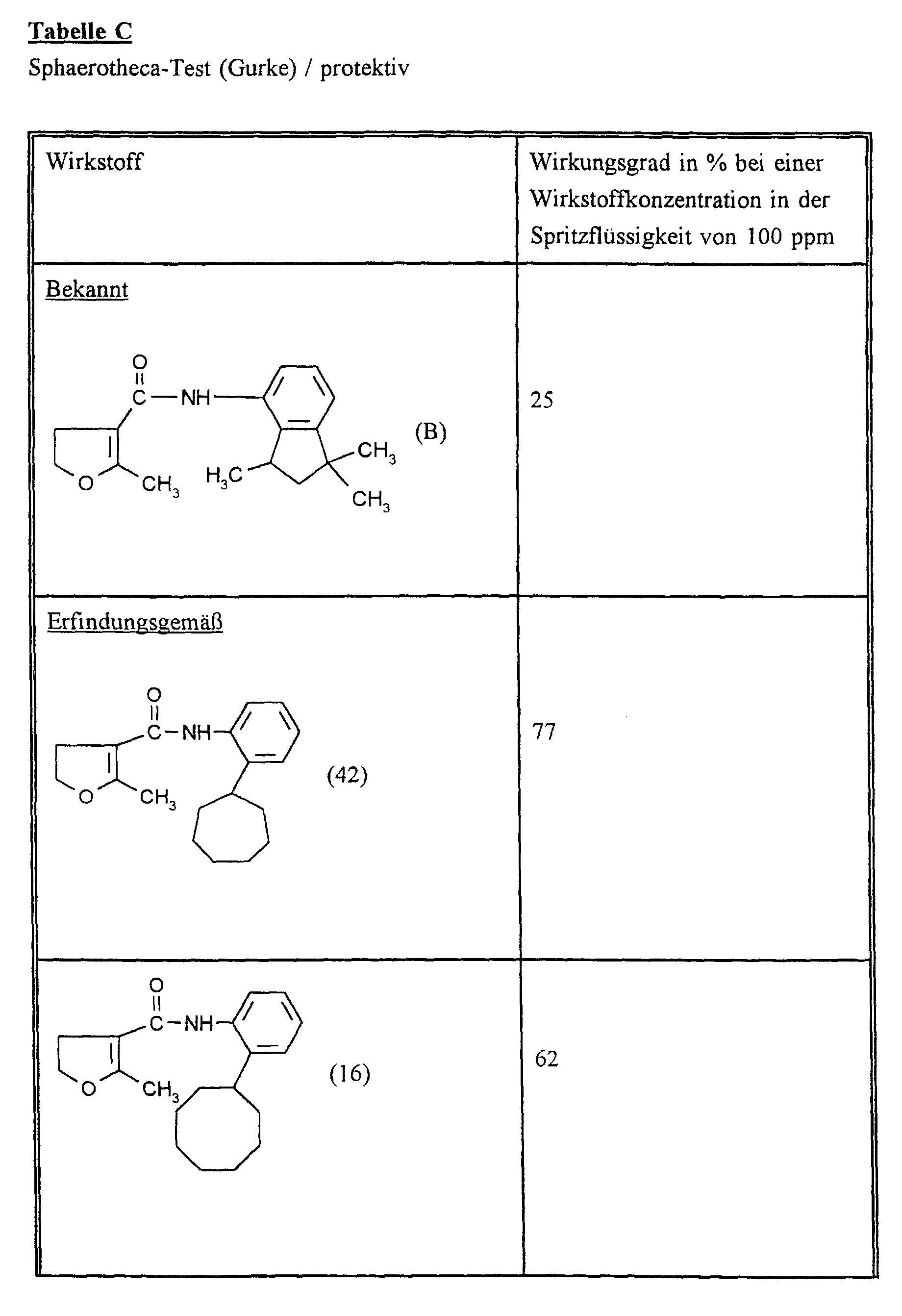

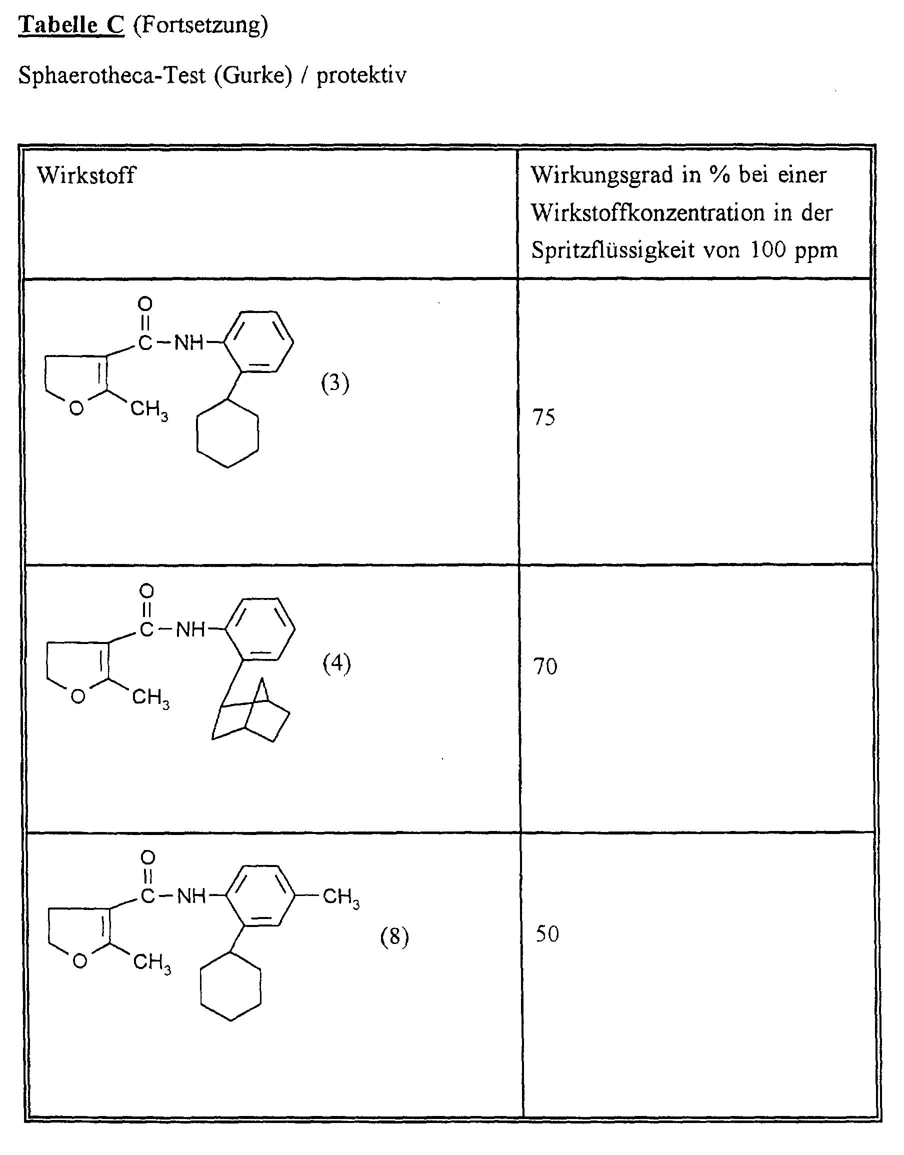

### Beispiel D

**Venturia-Test (Apfel)** / protektiv

| | |
|---|---|
| Lösungsmittel | 47 Gewichtsteile Aceton |
| Emulgator | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen mit einer wäßrigen Konidiensuspension des Apfelschorferregers Venturia inaequalis inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung. Dabei bedeutet 0 % ein Wirkungsgrad, der demjenigen der unbehandelten Kontrolle entspricht, während ein Wirkungsgrad von 100 % bedeutet, daß kein Befall beobachtet wird.

Wirkstoffe, Wirkstoffkonzentrationen und Versuchergebnisse gehen aus der folgenden Tabelle hervor.

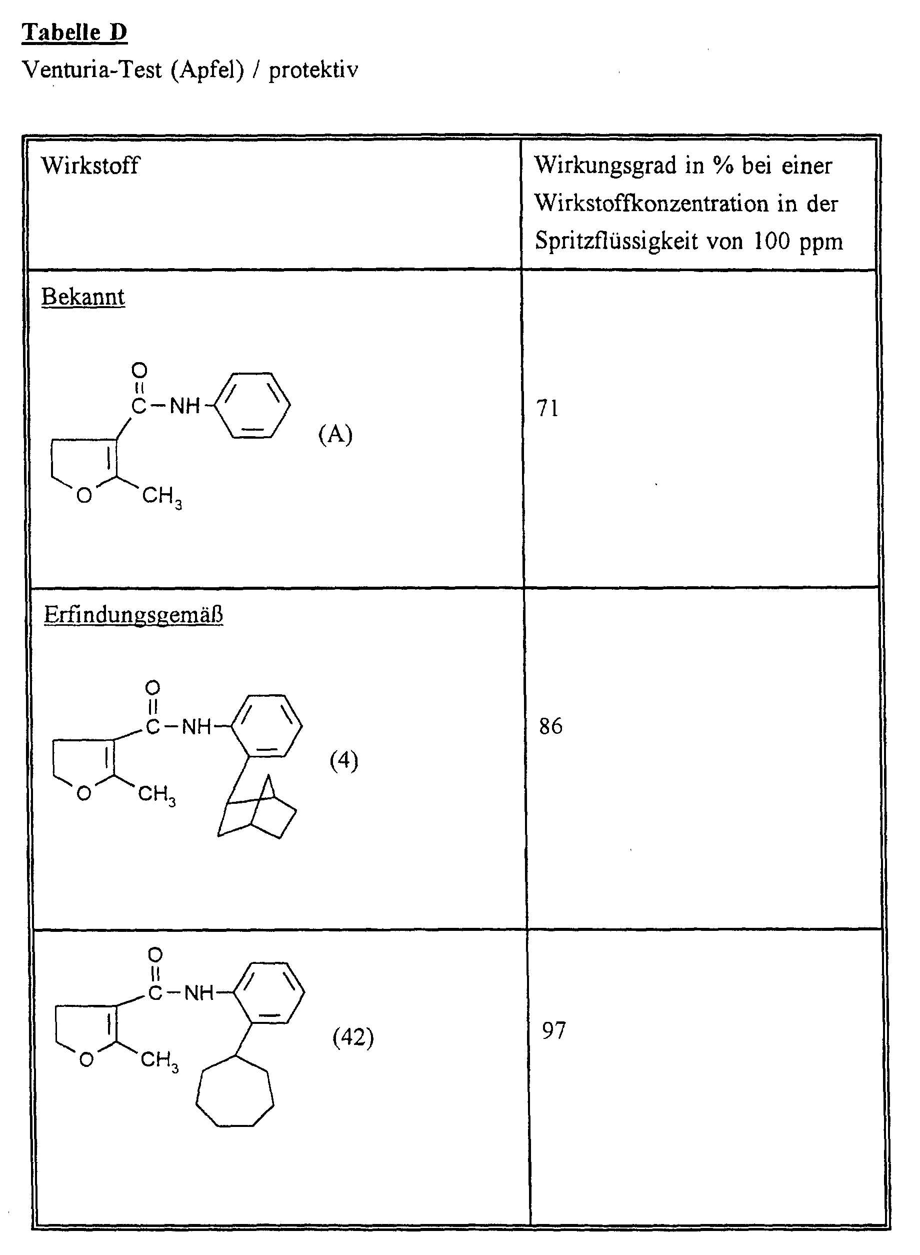

### Beispiel E

### Materialschutz-Test

### Hemmung des Wachstums von holzzerstörenden Basidiomyceten

### Lösungsmittel: Dimethylsulfoxid

Zur Herstellung einer zweckmäßigen Wirkstoffzubereitung werden 0,2 Gewichtsteile Wirkstoff mit 99,8 Gewichtsteilen des oben angegebenen Lösungsmittels versetzt.

Ein unter Verwendung von Malzextrakt-Pepton hergestellter Agar wird in flüssigem Zustand mit der Wirkstoffzubereitung in der jeweils gewünschten Aufwandmenge vermischt. Nach dem Aushärten wird der so hergestellte Nährstoffboden bei 27°C mit Mycelstücken inkubiert, die aus Kolonien von Basidiomyceten ausgestochen wurden.

Nach 3- bzw. 7-tägiger Lagerung bei 27°C erfolgt die Auswertung, indem das Hyphenwachstum gemessen und die im Vergleich zur unbehandelten Kontrolle aufgetretene Hemmung in Prozent bonitiert wird. Dabei bedeutet 0 % eine Wuchshemmung, die derjenigen der unbehandelten Kontrolle entspricht, während eine Wuchshemmung von 100 % bedeutet, daß kein Hyphenwachstum beobachtet wird.

Wirkstoffe, Wirkstoffkonzentrationen und Versuchsergebnisse gehen aus der folgenden Tabelle hervor.

**Tabelle E**

| Hemmung des Wachstums von holzzerstörenden Basidiomyceten | | |
|---|---|---|
| Pilzspezies | Prozentuale Hemmung des radialen Wachstums von Riesenkolonien bei 6 ppm an Wirkstoff gemäß Beispiel | |
| | (28) | (42) |
| Gloeophyllum trabeum | 50 | 60 |
| Coniophora puteana | 50 | 60 |
| Poria placenta | 30 | 50 |
| Lentinus tigrinus | 70 | 80 |
| Coriolus versicolor | 70 | 100 |
| Stereum sanguinolentum | 70 | 80 |

## Patentansprüche

1. Dihydrofuran-carboxamide der Formel 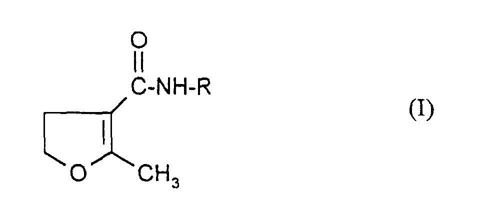 in welcher
R für Gruppierungen der Formeln 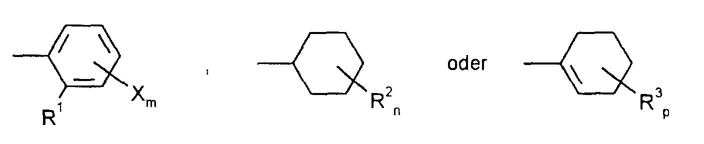 steht, worin
R¹ für gegebenenfalls substituiertes Cycloalkyl, gegebenenfalls substituiertes Bicycloalkyl, gegebenenfalls substituiertes Cycloalkenyl, gegebenenfalls substituiertes Cycloalkylalkyl, gegebenenfalls substituiertes Aryl, gegebenenfalls substituiertes Aroxy, gegebenenfalls substituiertes Aralkyl oder für Alkyl steht,
X für Alkyl mit 1 bis 6 Kohlenstoffatomen, Halogen, Cycloalkyl mit 3 bis 8 Kohlenstoffatomen, Alkoxy mit 1 bis 6 Kohlenstoffatomen, Halogenalkyl mit 1 bis 4 Kohlenstoffatomen und 1 bis 5 Halogenatomen oder Halogenalkoxy mit 1 bis 4 Kohlenstoffatomen und 1 bis 5 Halogenatomen steht,
m für ganze Zahlen von 0 bis 3 steht,
R² für Alkyl, Cycloalkyl, gegebenenfalls substituiertes Aryl oder gegebenenfalls substituiertes Aralkyl steht,
n für ganze Zahlen von 0 bis 3 steht,
R³ für Alkyl, Cycloalkyl, gegebenenfalls substituiertes Aryl oder gegebenenfalls substituiertes Aralkyl steht und
p für ganze Zahlen von 0 bis 3 steht.

2. Dihydrofuran-carboxamide der Formel (I) gemäß Anspruch 1, in denen
R für die Gruppierungen der Formeln 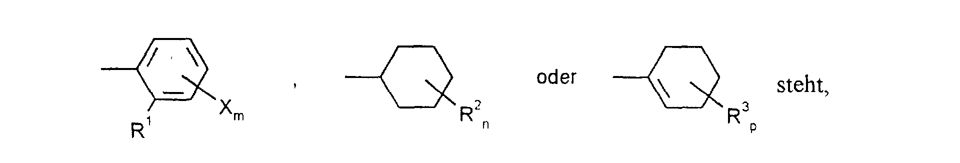 worin
R¹ für gegebenenfalls einfach bis dreifach, gleichartig oder verschieden durch Alkyl mit 1 bis 4 Kohlenstoffatomen substituiertes Cycloalkyl mit 3 bis 8 Kohlenstoffatomen, für gegebenenfalls einfach bis dreifach, gleichartig oder verschieden durch Alkyl mit 1 bis 4 Kohlenstoffatomen substituiertes Bicycloalkyl mit 7 bis 12 Kohlenstoffatomen, für gegebenenfalls einfach bis dreifach, gleichartig oder verschieden durch Alkyl mit 1 bis 4 Kohlenstoffatomen substituiertes Cycloalkenyl mit 5 bis 8 Kohlenstoffatomen oder für gegebenenfalls einfach bis dreifach, gleichartig oder verschieden durch Alkyl mit 1 bis 4 Kohlenstoffatomen substituiertes Cycloalkylalkyl mit 3 bis 8 Kohlenstoffatomen im Cycloalkylteil und 1 bis 4 Kohlenstoffatomen im Alkylteil steht, oder
für Phenyl steht, das einfach bis dreifach, gleichartig oder verschieden durch Halogen und/oder Alkyl mit 1 bis 4 Kohlenstoffatomen substituiert sein kann, oder
für Phenoxy steht, das einfach bis dreifach, gleichartig oder verschieden durch Halogen und/oder Alkyl mit 1 bis 4 Kohlenstoffatomen substituiert sein kann, oder
für Phenylalkyl mit 1 bis 4 Kohlenstoffatomen im Alkylteil steht, das im Phenylteil einfach bis dreifach, gleichartig oder verschieden durch Halogen und/oder Alkyl mit 1 bis 4 Kohlenstoffatomen substituiert sein kann,
oder für geradkettiges oder verzweigtes Alkyl mit 1 bis 12 Kohlenstoffatomen steht,
X für geradkettiges oder verzweigtes Alkyl mit 1 bis 4 Kohlenstoffatomen, Fluor, Chlor, Brom, Cycloalkyl mit 3 bis 8 Kohlenstoffatomen, geradkettiges oder verzweigtes Alkoxy mit 1 bis 4 Kohlenstoffatomen, Halogenalkyl mit 1 oder 2 Kohlenstoffatomen und 1 bis 5 Fluor-, Chlor- und/oder Bromatomen oder für Halogenalkoxy mit 1 oder 2 Kohlenstoffatomen und 1 bis 5 Fluor-, Chlor- und/oder Bromatomen steht,
m für die Zahlen 0, 1 oder 2 steht,
R² für geradkettiges oder verzweigtes Alkyl mit 1 bis 12 Kohlenstoffatomen, Cycloalkyl mit 3 bis 8 Kohlenstoffatomen, gegebenenfalls einfach bis dreifach, gleichartig oder verschieden durch Halogen und/oder Alkyl mit 1 bis 4 Kohlenstoffatomen substituiertes Phenyl oder für gegebenenfalls einfach bis dreifach, gleichartig oder verschieden durch Halogen und/oder Alkyl mit 1 bis 4 Kohlenstoffatomen substituiertes Phenylalkyl mit 1 bis 4 Kohlenstoffatomen im Alkylteil steht,
n für die Zahlen 0, 1, 2 oder 3 steht,
R³ für geradkettiges oder verzweigtes Alkyl mit 1 bis 12 Kohlenstoffatomen, Cycloalkyl mit 3 bis 8 Kohlenstoffatomen, gegebenenfalls einfach bis dreifach, gleichartig oder verschieden durch Halogen und/oder Alkyl mit 1 bis 4 Kohlenstoffatomen substituiertes Phenyl oder für gegebenenfalls einfach bis dreifach, gleichartig oder verschieden durch Halogen und/oder Alkyl mit 1 bis 4 Kohlenstoffatomen substituiertes Phenylalkyl mit 1 bis 4 Kohlenstoffatomen im Alkylteil steht und
p für die Zahlen 0, 1, 2 oder 3 steht.

3. Verfahren zur Herstellung von Dihydrofuran-carboxamiden der Formel (I) gemäß Anspruch 1, **dadurch gekennzeichnet, daß** man 2-Methyl-4,5-dihydrofuran-3-carbonsäure-halogenide der Formel 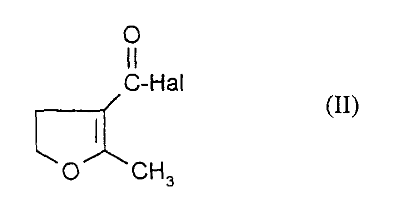 in welcher
Hal für Chlor oder Brom steht,
mit Aminen der Formel
H₂N-R (III)
in welcher
R die oben angegebenen Bedeutungen hat,
gegebenenfalls in Gegenwart eines Säurebindemittels und gegebenenfalls in Gegenwart eines Verdünnungsmittels umsetzt.

4. Mikrobizide Mittel, **gekennzeichnet durch** einen Gehalt an mindestens einem Dihydrofuran-carboxamid der Formel (I) gemäß Anspruch 1.

5. Verwendung von Dihydrofuran-carboxamiden der Formel (I) gemäß Anspruch 1 als Mikrobizide im Pflanzenschutz und im Materialschutz.

6. Verfahren zur Bekämpfung von unerwünschten Mikroorganismen im Pflanzenschutz und im Materialschutz, **dadurch gekennzeichnet, daß** man Dihydrofuran-carboxamide der Formel (I) gemäß Anspruch 1 auf die Mikroorganismen und/oder deren Lebensraum ausbringt.

7. Verfahren zur Herstellung von mikrobiziden Mitteln, **dadurch gekennzeichnet, daß** man Dihydrofuran-carboxamide der Formel (I) gemäß Anspruch 1 mit Streckmitteln und/oder oberflächenaktiven Stoffen vermischt.

## Claims

1. Dihydrofuran-carboxamides of the formula 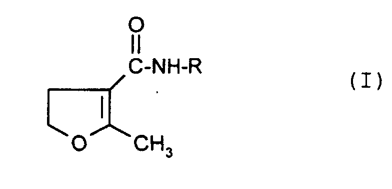 in which
R represents groupings of the formulae 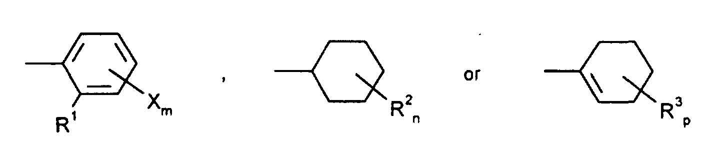 in which
R¹ represents optionally substituted cycloalkyl, optionally substituted bicycloalkyl, optionally substituted cycloalkenyl, optionally substituted cycloalkylalkyl, optionally substituted aryl, optionally substituted aroxy, optionally substituted aralkyl or represents alkyl,
X represents alkyl having 1 to 6 carbon atoms, halogen, cycloalkyl having 3 to 8 carbon atoms, alkoxy having 1 to 6 carbon atoms, halogenoalkyl having 1 to 4 carbon atoms and 1 to 5 halogen atoms or halogenoalkoxy having 1 to 4 carbon atoms and 1 to 5 halogen atoms,
m represents integers from 0 to 3,
R² represents alkyl, cycloalkyl, optionally substituted aryl or optionally substituted aralkyl,
n represents integers from 0 to 3,
R³ represents alkyl, cycloalkyl, optionally substituted aryl or optionally substituted aralkyl and
p represents integers from 0 to 3.

2. Dihydrofuran-carboxamides of the formula (I) according to Claim 1, in which
R represents the groupings of the formulae 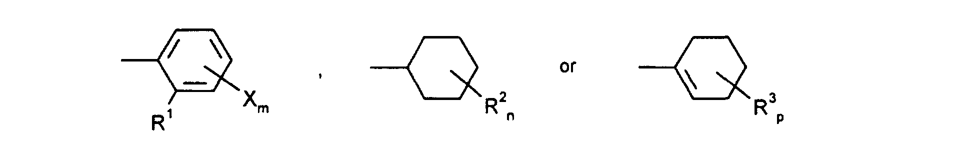 in which
R¹ represents cycloalkyl having 3 to 8 carbon atoms which is optionally mono-to trisubstituted by identical or different alkyl groups having 1 to 4 carbon atoms, represents bicycloalkyl having 7 to 12 carbon atoms which is optionally mono- to trisubstituted by identical or different alkyl groups having 1 to 4 carbon atoms, represents cycloalkenyl having 5 to 8 carbon atoms which is optionally mono- to trisubstituted by identical or different alkyl groups having 1 to 4 carbon atoms or represents cycloalkylalkyl having 3 to 8 carbon atoms in the cycloalkyl moiety and 1 to 4 carbon atoms in the alkyl moiety which is optionally mono-to trisubstituted by identical or different alkyl groups having 1 to 4 carbon atoms, or
represents phenyl which may be mono- to trisubstituted by identical or different substituents selected from the group consisting of halogen and alkyl having 1 to 4 carbon atoms, or
represents phenoxy which may be mono- to trisubstituted by identical or different substituents selected from the group consisting of halogen and alkyl having 1 to 4 carbon atoms, or
represents phenylalkyl having 1 to 4 carbon atoms in the alkyl moiety which may be mono- to trisubstituted in the phenyl moiety by identical or different substituents selected from the group consisting of halogen and alkyl having 1 to 4 carbon atoms, or represents straight-chain or branched alkyl having 1 to 12 carbon atoms,
X represents straight-chain or branched alkyl having 1 to 4 carbon atoms, fluorine, chlorine, bromine, cycloalkyl having 3 to 8 carbon atoms, straight-chain or branched alkoxy having 1 to 4 carbon atoms, halogenoalkyl having 1 or 2 carbon atoms and 1 to 5 fluorine, chlorine and/or bromine atoms or represents halogenoalkoxy having 1 or 2 carbon atoms and 1 to 5 fluorine, chlorine and/or bromine atoms,
m represents the numbers 0, 1 or 2,
R² represents straight-chain or branched alkyl having 1 to 12 carbon atoms, cycloalkyl having 3 to 8 carbon atoms, phenyl which is optionally mono- to trisubstituted by identical or different substituents selected from the group consisting of halogen and alkyl having 1 to 4 carbon atoms or represents phenylalkyl having 1 to 4 carbon atoms in the alkyl moiety which is optionally mono- to trisubstituted by identical or different substituents selected from the group consisting of halogen and alkyl having 1 to 4 carbon atoms,
n represents the numbers 0, 1, 2 or 3,
R³ represents straight-chain or branched alkyl having 1 to 12 carbon atoms, cycloalkyl having 3 to 8 carbon atoms, phenyl which is optionally mono- to trisubstituted by identical or different substituents selected from the group consisting of halogen and alkyl having 1 to 4 carbon atoms or represents phenylalkyl having 1 to 4 carbon atoms in the alkyl moiety which is optionally mono- to trisubstituted by identical or different substituents selected from the group consisting of halogen and alkyl having 1 to 4 carbon atoms and
p represents the numbers 0, 1, 2 or 3.

3. Process for preparing dihydrofuran-carboxamides of the formula (I) according to Claim 1, **characterized in that** 2-methyl-4,5-dihydrofuran-3-carbonyl halides of the formula 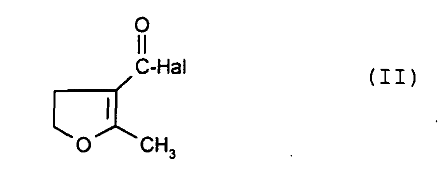 in which
Hal represents chlorine or bromine,
are reacted with amines of the formula
H₂N-R (III)
in which
R is as defined above,
if appropriate in the presence of an acid binder and if appropriate in the presence of a diluent.

4. Microbicidal compositions, **characterized in that** they contain at least one dihydrofuran-carboxamide of the formula (I) according to Claim 1.

5. Use of dihydrofuran-carboxamides of the formula (I) according to Claim 1 as microbicides in crop protection and in the protection of materials.

6. Method for controlling undesirable microorganisms in crop protection and in the protection of materials, **characterized in that** dihydrofuran-carboxamides of the formula (I) according to Claim 1 are applied to the microorganisms and/or their habitat.

7. Process for preparing microbicidal compositions, **characterized in that** dihydrofuran-carboxamides of the formula (I) according to Claim 1 are mixed with extenders and/or surfactants.

## Revendications

1. Dihydrofuranne-carboxamides de formule 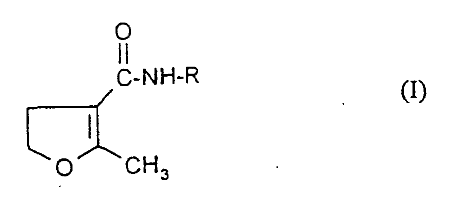 dans laquelle
R représente les groupements de formules 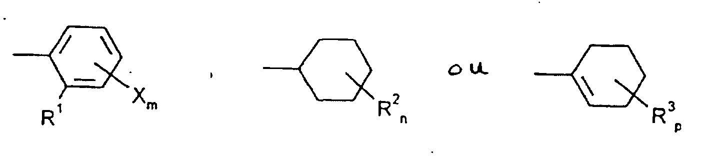 où
R¹ désigne un reste cycloalkyle éventuellement substitué, un reste bicycloalkyle éventuellement substitué, un reste cyclo-alcényle éventuellement substitué, un reste cycloalkylalkyle éventuellement substitué, un reste aryle éventuellement substitué, un reste aroxy éventuellement substitué, un reste aralkyle éventuellement substitué ou un reste alkyle,
X est un reste alkyle ayant 1 à 6 atomes de carbone, un halogène, un reste cycloalkyle ayant 3 à 8 atomes de carbone, un reste alkoxy ayant 1 à 6 atomes de carbone, un reste halogénalkyle ayant 1 à 4 atomes de carbone et 1 à 5 atomes d'halogène ou un reste halogénalkoxy ayant 1 à 4 atomes de carbone et 1 à 5 atomes d'halogène,
m représente des nombres entiers de 0 à 3,
R² est un reste alkyle, cycloalkyle, aryle éventuellement substitué ou aralkyle éventuellement substitué,
n représente des nombres entiers de 0 à 3,
R³ est un reste alkyle, cycloalkyle, aryle éventuellement substitué ou aralkyle éventuellement substitué et
p représente des nombres entiers de 0 à 3.

2. Dihydrofuranne-carboxamides de formule (I) suivant la revendication 1, dans lesquels
R représente les groupements de formules 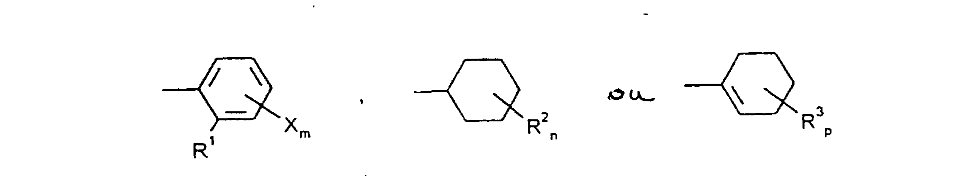 où
R¹ est un reste cycloalkyle ayant 3 à 8 atomes de carbone éventuellement substitué une à trois fois identiques ou différentes par un radical alkyle ayant 1 à 4 atomes de carbone, un reste bicycloalkyle ayant 7 à 12 atomes de carbone éventuellement substitué une à trois fois identiques ou différentes par un radical alkyle ayant 1 à 4 atomes de carbone, un reste cycloalcényle ayant 5 à 8 atomes de carbone éventuellement substitué une à trois fois identiques ou différentes par un radical alkyle ayant 1 à 4 atomes de carbone ou un reste cycloalkylalkyle ayant 3 à 8 atomes de carbone dans la partie cycloalkyle et 1 à 4 atomes de carbone dans la partie alkyle, éventuellement substitué une à trois fois identiques ou différentes par un radical alkyle ayant 1 à 4 atomes de carbone, ou bien un reste phényle qui peut être substitué une à trois fois identiques ou différentes par un halogène et/ou par un radical alkyle ayant 1 à 4 atomes de carbone,
un reste phénoxy qui peut être substitué une à trois fois identiques ou différentes par un halogène et/ou par un radical alkyle ayant 1 à 4 atomes de carbone, ou bien
un reste phénylalkyle ayant 1 à 4 atomes de carbone dans la partie alkyle, qui peut être substitué dans la partie phényle une à trois fois identiques ou différentes par un halogène et/ou par un radical alkyle ayant 1 à 4 atomes de carbone, ou bien
un reste alkyle linéaire ou ramifié ayant 1 à 12 atomes de carbone,
X est un reste alkyle linéaire ou ramifié ayant 1 à 4 atomes de carbone, le fluor, le chlore, le brome, un reste cycloalkyle ayant 3 à 8 atomes de carbone, un reste alkoxy linéaire ou ramifié ayant 1 à 4 atomes de carbone, un reste halogénalkyle ayant 1 ou 2 atomes de carbone et 1 à 5 atomes de fluor, de chlore et/ou de brome ou un reste halogénalkoxy ayant 1 ou 2 atomes de carbone et 1 à 5 atomes de fluor, de chlore et/ou de brome,
m représente les nombres 0, 1 ou 2,
R² est un reste alkyle linéaire ou ramifié ayant 1 à 12 atomes de carbone, un reste cycloalkyle ayant 3 à 8 atomes de carbone, un reste phényle éventuellement substitué une à trois fois identiques ou différentes par un halogène et/ou par un radical alkyle ayant 1 à 4 atomes de carbone ou un reste phénylalkyle ayant 1 à 4 atomes de carbone dans la partie alkyle, éventuellement substitué une à trois fois identiques ou différentes par un halogène et/ou par un radical alkyle ayant 1 à 4 atomes de carbone,
n représente les nombres 0, 1, 2 ou 3,
R³ est un reste alkyle linéaire ou ramifié ayant 1 à 12 atomes de carbone, un reste cycloalkyle ayant 3 à 8 atomes de carbone, un reste phényle éventuellement substitué une à trois fois identiques ou différentes par un halogène et/ou par un radical alkyle ayant 1 à 4 atomes de carbone, ou un reste phénylalkyle ayant 1 à 4 atomes de carbone dans la partie alkyle, éventuellement substitué une à trois fois identiques ou différentes par un halogène et/ou par un radical alkyle ayant 1 à 4 atomes de carbone, et
p représente les nombres 0, 1, 2 ou 3.

3. Procédé de production de dihydrofuranne-carboxamides de formule (I) suivant la revendication 1, **caractérisé en ce qu'**on fait réagir des halogénures d'acide 2-méthyl-4,5-dihydrofuranne-3-carboxylique de formule 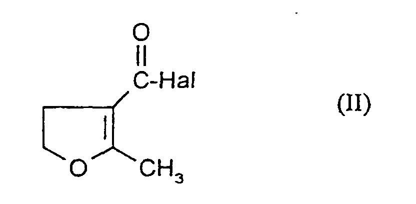 dans laquelle
Hal représente le chlore ou le brome,
avec des amines de formule
H₂N-R (III)
dans laquelle
R a les définitions indiquées ci-dessus,
le cas échéant en présence d'un accepteur d'acide et en présence éventuelle d'un diluant.

4. Compositions microbicides, **caractérisées par** une teneur en au moins un dihydrofuranne-carboxamide de formule (I) suivant la revendication 1.

5. Utilisation de dihydrofuranne-carboxamides de formule (I) suivant la revendication 1 comme microbicides dans la protection des plantes et dans la protection des matériaux.

6. Procédé pour combattre des micro-organismes indésirables dans la protection des plantes et dans la protection des matériaux, **caractérisé en ce qu'**on épand des dihydrofuranne-carboxamides de formule (I) suivant la revendication 1 sur les micro-organismes et/ou sur leur milieu.

7. Procédé de préparation de compositions microbicides, **caractérisé en ce qu'**on mélange des dihydrofuranne-carboxamides de formule (I) suivant la revendication 1 avec des diluants et/ou des agents tensioactifs.
